# EUROPEAN PATENT APPLICATION

(11) **EP 2 283 852 A2**
(43) Date of publication of application: **16.02.2011**
(21) Application number: 10185466.9
(22) Date of filing: 10.12.1998
(51) Int. Cl.: A61K 38/16, C07K 14/195, C12N 15/31, C12Q 1/68, A61K 39/00, A61P 1/02

(54) **Porphorymonas gingivalis polypeptides and polynucleotides**

(30) Priority: 10.12.1997 AU PP083997; 31.12.1997 AU PP118297; 30.01.1998 AU PP154698; 10.03.1998 AU PP226498; 09.04.1998 AU PP291198; 23.04.1998 AU PP312898; 05.05.1998 AU PP333898; 22.05.1998 AU PP365498; 29.07.1998 AU PP491798; 30.07.1998 AU PP496398; 04.08.1998 AU PP502898
(62) Divisional of application: 08100586.0
(71) Applicant: CSL Limited, Parkville, Victoria 3052 (AU); The University of Melbourne, Melbourne, VIC 3052 (AU)
(72) Inventor: Ross, Bruce Carter, Coburg, Victoria 3058 (AU); Barr, Ian George, Templestowe, Victoria 3106 (AU); Patterson, Michelle Anne, Laverton, Victoria 3028 (AU); Agius, Catherine Therese, Box Hill South, Victoria 3128 (AU); Rothel, Linda Joy, Glen Huntly, Victoria 3163 (AU); Margetts, Mai Brigid, Moonee Ponds, Victoria 3039 (AU); Hocking, Dianna Margaret, Flemington, Victoria 3031 (AU); Webb, Elizabeth Ann, Eltham, Victoria 3422 (AU)
(74) Representative: Lee, Nicholas John

(57) **Abstract**

The present invention relates to isolated *Porphorymonas gingivalis* polypeptides and nucleotides. The polypeptides include;
an amino acid sequence selected from the group consisting of SEQ. ID. NO. 265 to SEQ. ID. NO. 528, SEQ. ID. NO. 531 and SEQ. ID. NO. 532; or
an amino acid sequence at least 85%, preferably at least 95%, identical to an amino acid sequence selected from the group consisting of SEQ. ID. NO. 265 to SEQ. ID. NO. 528, SEQ. ID. NO. 531 and SEQ. ID. NO. 532; or
at least 40 amino acids having a contiguous sequence of at least 40 amino acids identical to a contiguous amino acid sequence selected from the group consisting of SEQ. ID. NO. 265 to SEQ. ID. NO. 528, SEQ. ID. NO. 531 and SEQ. ID. NO. 532.

## Description

### FIELD OF THE INVENTION

The present invention relates to *P. gingivalis* nucleotide sequences, *P. gingivalis* polypeptides and probes for detection of *P. gingivalis.* The *P. gingivalis* polypeptides and nucleotides can be used in compositions for use in raising an immune response in a subject against *P. gingivalis* and treating or preventing or reducing the severity of the condition known as periodontitis.

### BACKGROUND OF THE INVENTION

Periodontal diseases are bacterial-associated inflammatory diseases of the supporting tissues of the teeth and range from the relatively mild form of gingivitis, the non-specific, reversible inflammation of gingival tissue to the more aggressive forms of periodontitis which are characterised by the destruction of the tooth's supporting structures. Periodontitis is associated with a subgingival infection of a consortium of specific Gram-negative bacteria that leads to the destruction of the periodontium and is a major public health problem. One bacterium that has attracted considerable interest is *P. gingivalis* as the recovery of this microorganism from adult periodontitis lesions can be up to 50% of the subgingival anaerobically cultivable flora, whereas *P. gingivalis* is rarely recovered, and then in low numbers, from healthy sites. A proportional increase in the level of *P. gingivalis* in subgingival plaque has been associated with an increased severity of periodontitis and eradication of the microorganism from the cultivable subgingival microbial population is accompanied by resolution of the disease. The progression of periodontitis lesions in non-human primates has been demonstrated with the subgingival implantation of *P. gingivalis.* These findings in both animals and humans suggest a major role for *P. gingivalis* in the development of adult periodontitis.

*P. gingivalis* is a black-pigmented, anaerobic, asaccharolytic, proteolytic Gram-negative rod that obtains energy from the metabolism of specific amino acids. The microorganism has an absolute growth requirement for iron, preferentially in the form of haeme or its Fe(III) oxidation product haemin and when grown under conditions of excess haemin is highly virulent in experimental animals. A number of virulence factors have been implicated in the pathogenicity of *P. gingivalis* including the capsule, adhesins, cytotoxins and extracellular hydrolytic enzymes.

In order to develop an efficacious and safe vaccine to prevent, eliminate or reduce *P. gingivalis* colonisation it is necessary to identify and produce antigens that are involved in virulence that have utility as immunogens possibly through the generation of specific antibodies. Whilst it is possible to attempt to isolate antigens directly from cultures of *P. gingivalis* this is often difficult. For example as mentioned above, *P. gingivalis* is a strict anaerobe and can be difficult to isolate and grow. It is also known that, for a number of organisms, when cultured *in vitro* that many virulence genes are down regulated and the encoded proteins are no longer expressed. If conventional chemistry techniques were applied to purify vaccine candidates potentially important (protective) molecules may not be identified. With DNA sequencing, as the gene is present (but not transcribed) even when the organism is grown *in vitro* it can be identified, cloned and produced as a recombinant DNA protein. Similarly, a protective antigen or therapeutic target may be transiently expressed by the organism *in vitro* or produced in low levels making the identification of these molecules extremely difficult by conventional methods.

With serological identification of therapeutic targets one is limited to those responses which are detectable using standard methods such as Western Blotting or ELISA. The limitation here is the both the level of response that is generated by the animal or human and determining whether this response is protective, damaging or irrelevant. No such limitation is present with a sequencing approach to the identification of potential therapeutic or prophylactic targets.

It is also well known that *P. gingivalis* produces a range of broadly active proteases (University of Melbourne International Patent Application No PCT /AU 96/00673, US Patent Nos 5,475,097 and 5,523,390), which make the identification of intact proteins difficult because of their degradation by these proteases.

### SUMMARY OF THE INVENTION

The present inventors have attempted to isolate *P. gingivalis* nucleotide sequences which can be used for recombinant production of *P. gingivalis* polypeptides and to develop nucleotide probes specific for *P. gingivalis.* The DNA sequences listed below have been selected from a large number of *P. gingivalis* sequences according to their indicative potential as vaccine candidates. This intuitive step involved comparison of the deduced protein sequence from the *P. gingivalis* DNA sequences to the known protein sequence databases. Some of the characteristics used to select useful vaccine candidates include; the expected cellular location, such as outer membrane proteins or secreted proteins, particular functional activities of similar proteins such as those with an enzymatic or proteolytic activity, proteins involved in essential metabolic pathways that when inactivated or blocked may be deleterious or lethal to the organism, proteins that might be expected to play a role in the pathogenesis of the organism eg. red cell lysis, cell agglutination or cell receptors and proteins which are paralogues to proteins with proven vaccine efficacy.

In a first aspect the present invention consists an isolated antigenic *Porphorymonas gingivalis* polypeptide, the polypeptide comprising;
an amino acid sequence selected from the group consisting of SEQ. ID. NO. 265 to SEQ. ID. NO. 528, SEQ. ID. NO. 531 and SEQ. ID. NO. 532; or
an amino acid sequence at least 85%, preferably at least 95%, identical to an amino acid sequence selected from the group consisting of SEQ. ID. NO. 265 to SEQ. ID. NO. 528, SEQ. ID. NO. 531 and SEQ. ID. NO. 532; or
at least 40 amino acids having a contiguous sequence of at least 40 amino acids identical to a contiguous amino acid sequence selected from the group consisting of SEQ. ID. NO. 265 to SEQ. ID. NO. 528, SEQ. ID. NO. 531 and SEQ. ID. NO. 532.

In an embodiment of the present invention the polypeptide comprises;
an amino acid sequence selected from the group consisting of SEQ. ID. NO. 386 to SEQ. ID. NO. 528 and SEQ. ID. NO. 532; or
an amino acid sequence at least 85%, preferably at least 95%, identical to an amino acid sequence selected from the group consisting of SEQ. ID. NO. 386 to SEQ. ID. NO. 528 and SEQ. ID. NO. 532; or
at least 40 amino acids having a contiguous sequence of at least 40 amino acids identical to a contiguous amino acid sequence selected from the group consisting of SEQ. ID. NO. 386 to SEQ. ID. NO. 528 and SEQ. ID. NO. 532.

As used herein % identity for polypeptides is to be calculated using the alignment algorithm of Needleman and Munsch (9) using a standard protein scoring matrix (Blosum 50).

In a preferred embodiment of the present invention the polypeptide comprises an amino acid sequence of SEQ. ID. NO. 526 or SEQ. ID. NO. 383.

In a second aspect the present invention consists in an isolated antigenic *Porphorymonas gingivalis* polypeptide, the polypeptide comprising an amino acid sequence selected from the group consisting of SEQ. ID. NO. 386 to SEQ. ID. NO. 528 and SEQ. ID. NO. 532 less the leader sequence set out in Table 3.

In a third aspect the present invention consists in an isolated DNA molecule, the DNA molecule comprising a nucleotide sequence which encodes the polypeptide of the first aspect the present invention or a sequence which hybridises thereto under stringent conditions.

It is preferred that the isolated DNA molecule comprises a nucleotide sequence selected from the group consisting of SEQ. ID. NO. 1 to SEQ. ID. NO. 264, SEQ. ID. NO. 529 and SEQ. ID. NO. 530.

In a preferred embodiment of the third aspect of the present invention the DNA molecule comprises a nucleotide sequence of SEQ. ID. NO. 119 or SEQ. ID. NO. 262.

In a fourth aspect the present invention consists in a recombinant expression vector comprising the DNA molecule of the second aspect of the present invention operably linked to a transcription regulatory element.

The present invention also provides a cell comprising this recombinant expression vector.

In a further aspect the present invention consists in a method for producing a *P. gingivalis* polypeptide comprising culturing the cell under conditions that permit expression of the polypeptide.

In yet a further aspect the present invention provides a composition for use in raising an immune response directed against *P. gingivalis* in a subject, the composition comprising an effective amount of at least one polypeptide of the first aspect of the present invention, or at least one DNA molecule of the second aspect of the present invention, or both,and a pharmaceutically acceptable carrier. It is preferred that the pharmaceutically acceptable carrier is an adjuvant. In other aspects the present invention provides methods of treating *P. gingivalis* infection in subject comprising the administration of the composition to the subject such that treatment of *P. gingivalis* infection occurs. The treatment may be prophylactic or therapeutic.

In yet another aspect the present invention provides an antibody raised against a polypeptide of the first aspect the invention. The antibody may be polyclonal or monoclonal. The present invention also provides compositions including these antibodies. It is preferred that these compositions are adapted for oral use and may be, for example, dentrifices, mouthwashes, etc.

In a still further aspect the present invention provides a nucleotide probe comprising at least 18 nucleotides and having a contiguous sequence of at least 18 nucleotides identical to a contiguous nucleotide sequence selected from the group consisting of SEQ. ID. NO. 1 to SEQ. ID. NO. 121, SEQ. ID. NO. 529, and sequences complementary thereto. It is preferred that the probe further comprises a detectable label.

The present invention also provides a method for detecting the presence of *P. gingivalis* nucleic acid in a sample comprising:
(a) contacting a sample with the nucleotide probe under conditions in which a hybrid can form between the probe and a *P. gingivalis* nucleic acid in the sample; and
(b) detecting the hybrid formed in step (a), wherein detection of a hybrid indicates the presence of a *P. gingivalis* nucleic acid in the sample.

### DETAILED DESCRIPTION

### Definitions

A purified or isolated polypeptide or a substantially pure preparation of a polypeptide are used interchangeably herein and, as used herein, mean a polypeptide that has been separated from other proteins, lipids, and nucleic acids with which it naturally occurs. Preferably, the polypeptide is also separated from substances, e.g., antibodies or gel matrix, e.g., polyacrylamide, which are used to purify it. Preferably, the polypeptide constitutes at least 10, 20, 50 70, 80 or 95% dry weight of the purified preparation. Preferably, the preparation contains: sufficient polypeptide to allow protein sequencing; at least 1, 10, or 100 mg of the polypeptide.

A purified preparation of cells refers to, in the case of plant or animal cells, an in vitro preparation of cells and not an entire intact plant or animal. In the case of cultured cells or microbial cells, it consists of a preparation of at least 10% and more preferably 50% of the subject cells.

A purified or isolated or a substantially pure nucleic acid, e.g., a substantially pure DNA, (are terms used interchangeably herein) is a nucleic acid which is one or both of the following: not immediately contiguous with both of the coding sequences with which it is immediately contiguous (i.e., one at the 5' end and one at the 3' end) in the naturally occurring genome of the organism from which the nucleic acid is derived; or which is substantially free of a nucleic acid with which it occurs in the organism from which the nucleic acid is derived. The term includes, for example, a recombinant DNA which is incorporated into a vector, e.g., into an autonomously replicating plasmid or virus, or into the genomic DNA of a prokaryote or eukaryote, or which exists as a separate molecule (e.g., a cDNA or a genomic DNA fragment produced by PCR or restriction endonuclease treatment) independent of other DNA sequences. Substantially pure DNA also includes a recombinant DNA which is part of a hybrid gene encoding additional *P. gingivalis* DNA sequence.

A "contig" as used herein is a nucleic acid representing a continuous stretch of genomic sequence of an organism.

An "open reading frame", also referred to herein as ORF, is a region of nucleic acid which encodes a polypeptide. This region may represent a portion of a coding sequence or a total sequence and can be determined from a stop to stop codon or from a start to stop codon.

As used herein, a "coding sequence" is a nucleic acid which is transcribed into messenger RNA and/or translated into a polypeptide when placed under the control of appropriate regulatory sequences. The boundaries of the coding sequence are determined by a translation start codon at the five prime terminus and a translation stop code at the three prime terminus. A coding sequence can include but is not limited to messenger RNA synthetic DNA, and recombinant nucleic acid sequences.

A "complement" of a nucleic acid as used herein refers to an anti-parallel or antisense sequence that participates in Watson-Crick base-pairing with the original sequence.

A "gene product" is a protein or structural RNA which is specifically encoded by a gene.

As used herein, the term "probe" refers to a nucleic acid, peptide or other chemical entity which specifically binds to a molecule of interest. Probes are often associated with or capable of associating with a label. A label is a chemical moiety capable of detection. Typical labels comprise dyes, radioisotopes, luminescent and chemiluminescent moieties, fluorophores, enzymes, precipitating agents, amplification sequences, and the like. Similarly, a nucleic acid, peptide or other chemical entity which specifically binds to a molecule of interest and immobilizes such molecule is referred herein as a "capture ligand". Capture ligands are typically associated with or capable of associating with a support such as nitro-cellulose, glass, nylon membranes, beads, particles and the like. The specificity of hybridization is dependent on conditions such as the base pair composition of the nucleotides, and the temperature and salt concentration of the reaction. These conditions are readily discernible to one of ordinary skill in the art using routine experimentation.

Homologous refers to the sequence similarity or sequence identity between two polypeptides or between two nucleic acid molecules. When a position in both of the two compared sequences is occupied by the same base or amino acid monomer subunit, e.g., if a position in each of two DNA molecules is occupied by adenine, then the molecules are homologous at that position. The percent of homology between two sequences is a function of the number of matching or homologous positions shared by the two sequences divided by the number of positions compared x 100.

The terms peptides, proteins, and polypeptides are used interchangeably herein.

An "immunogenic component" as used herein is a moiety, such as an *P. gingivalis* polypeptide, analog or fragment thereof, that is capable of eliciting a humoral and/or cellular immune response in a host animal.

An "antigenic component" as used herein is a moiety, such as *P. gingivalis* polypeptide, analog or fragment thereof, that is capable of binding to a specific antibody with sufficiently high affinity to form a detectable antigen-antibody complex.

As used herein, the term "cell-specific promoter" means a DNA sequence that serves as a promoter, i.e., regulates expression of a selected DNA sequence operably linked to the promoter, and which effects expression of the selected DNA sequence in specific cells of a tissue. The term also covers so-called "leaky" promoters, which regulate expression of a selected DNA primarily in one tissue, but cause expression in other tissues as well.

As used herein, the term "control sequence" refers to a nucleic acid having a base sequence which is recognized by the host organism to effect the expression of encoded sequences to which they are ligated. The nature of such control sequences differs depending upon the host organism; in prokaryotes, such control sequences generally include a promoter, ribosomal binding site, terminators, and in some cases operators; in eukaryotes, generally such control sequences include promoters, terminators and in some instances, enhancers. The term control sequence is intended to include at a minimum, all components whose presence is necessary for expression, and may also include additional components whose presence is advantageous, for example, leader sequences.

As used herein, the term "operably linked" refers to sequences joined or ligated to function in their intended manner. For example, a control sequence is operably linked to coding sequence by ligation in such a way that expression of the coding sequence is achieved under conditions compatible with the control sequence and host cell.

A "sample" as used herein refers to a biological sample, such as, for example, tissue or fluid isolated from an individual (including without limitation plasma. serum, cerebrospinal fluid, lymph, tears, saliva and tissue sections) or from *in vitro* cell culture constituents, as well as samples from the environment.

The practice of the invention will employ, unless otherwise indicated, conventional techniques of chemistry, molecular biology, microbiology, recombinant DNA, and immunology well known to those skilled in the art. Such techniques are described and explained throughout the literature in sources such as, J. Perbal, A Practical Guide to Molecular Cloning, John Wiley and Sons (1984), J. Sambrook et al., Molecular Cloning: A Laboratory Manual, Cold Spring Harbour Laboratory Press (1989), T.A. Brown (editor), Essential Molecular Biology: A Practical Approach, Volumes 1 and 2, IRL Press (1991), D.M. Glover and B.D. Hames (editors), DNA Cloning: A Practical Approach, Volumes 1-4, IRL Press (1995 and 1996), and F.M. Ausubel et al. (Editors), Current Protocols in Molecular Biology, Greene Pub. Associates and Wiley-Interscience (1988, including all updates until present). The disclosure of these texts are incorporated herein by reference.

### Pharmaceutically Acceptable Carriers

The antibodies, polypeptides and DNA of the present invention can be included in compositions which include a carrier or diluent. These compositions include pharmaceutical compositions where the carrier or diluent will be pharmaceutically acceptable. Pharmaceutically acceptable carriers or diluents include those used in compositions suitable for oral, rectal, nasal, topical (including buccal and sublingual), vaginal, parenteral (including subcutaneous, intramuscular, intravenous, intradermal, intrathecal and epidural) administration. They are nontoxic to recipients at the dosages and concentrations employed. Representative examples of pharmaceutically acceptable carriers or diluents include, but are not limited to; water, isotonic solutions which are preferably buffered at a physiological pH (such as phosphate-buffered saline or Tris-buffered saline) and can also contain one or more of, mannitol, lactose, trehalose, dextrose, glycerol, ethanol or polypeptides (such as human serum albumin). The compositions may conveniently be presented in unit dosage form and may be prepared by any of the methods well known in the art of pharmacy.

As will be well understood by those skilled in the art alterations may be made to the amino acid sequences set out in the Sequence Listings. These alterations may be deletions, insertions, or substitutions of amino acid residues. The altered polypeptides can be either naturally occurring (that is to say, purified or isolated from a natural source) or synthetic (for example, by performing site-directed metagenesis on the encoding DNA). It is intended that such altered polypeptides which have at least 85%, preferably at least 95% identity with the sequences set out in the Sequence Listing are within the scope of the present invention. Antibodies raised against these altered polypeptides will also bind to the polypeptides having one of the sequences set out in the Sequence Listings. The level of % identity is to be calculated as set out above.

Protein sequences are homologous if they are related by divergence from a common ancestor. Consequently, a species homologue of the protein will be the equivalent protein which occurs naturally in another species. Within any one species a homologue may exist as numerous allelic variants, and these will be considered homologues of the protein. Allelic variants and species homologues can be obtained by following standard techniques known to those skilled in the art.

An allelic variant will be a variant that is naturally occurring within an individual organism.

### Mutants, Variants and Homology - Nucleic Acids

Mutant polynucleotides will possess one or more mutations which are deletions, insertions, or substitutions of nucleotide residues. Mutants can be either naturally occurring (that is to say, isolated from a natural source) or synthetic (for example, by performing site-directed metagenesis on the DNA). It is thus apparent that polynucleotides of the invention can be either naturally occurring or recombinant (that is to say prepared using recombinant DNA techniques).

An allelic variant will be a variant that is naturally occurring within an individual organism.

Nucleotide sequences are homologous if they are related by divergence from a common ancestor. Consequently, a species homologue of the polynucleotide will be the equivalent polynucleotide which occurs naturally in another species. Within any one species a homologue may exist as numerous allelic variants, and these will be considered homologues of the polynucleotide. Allelic variants and species homologues can be obtained by following standard techniques known to those skilled in the art.

### Antibody Production

Antibodies, either polyclonal or monoclonal, which are specific for a polypeptide of the present invention can be produced by a person skilled in the art using standard techniques such as, but not limited to, those described by Harlow et al. Antibodies: A Laboratory Manual, Cold Springs Harbor Laboratory Press (1988), and D. Catty (editor), Antibodies: A Practical Approach, IRL Press (1988).

Various procedures known in the art may be used for the production of polyclonal antibodies to epitopes of a protein. For the production of polyclonal antibodies, a number of host animals are acceptable for the generation of antibodies by immunization with one or more injections of a polypeptide preparation, including but not limited to rabbits, mice, rats, etc. Various adjuvants may be used to increase the immunological response in the host animal, depending on the host species, including but not limited to Freund's (complete and incomplete), mineral gels such as aluminium hydroxide, surface active substances such as lysolecithin, pluronic polyols, polyanions, oil emulsions, keyhole lympet hemocyanins, dinitrophenol, and potentially useful human adjuvants such as BCG (bacille Calmette-Guerin) and Corynebacterium parvum.

A monoclonal antibody to an epitope of a protein may be prepared by using any technique which provides for the production of antibody molecules by continuous cell lines in culture. These include but are not limited to the hybridoma technique originally described by Kohler and Milstein (1975, Nature 256, 493-497), and the more recent human B-cell hybridoma technique (Kesber et al. 1983, Immunology Today 4:72) and EBV-hybridoma technique (Cole et al. 1985, Monoclonal Antibodies and Cancer Therapy, Alan R. Liss, Inc. pp. 77-96). In addition, techniques developed for the production of "chimeric antibodies" by splicing the genes from antibody molecule of appropriate antigen specificity together with genes from a human antibody molecule of appropriate biological activity may be used (Morrison et al. 1984, Proc. Natl. Acad. Sci., 81:6851-6855; Neuberger et al. 1984 Nature 312:604-608; Takeda et al. 1985 Nature 31:452-454). Alternatively, techniques described for the production of single chain antibodies (U.S. Patent 4,946,778) can be adapted to produce 4-specific single chain antibodies.

Recombinant human or humanized versions of monoclonal antibodies are a preferred embodiment for human therapeutic applications. Humanized antibodies may be prepared according to procedures in the literature (e.g. Jones et al. 1986, Nature 321:522-25; Reichman et al. 1988 Nature 332:323-27; Verhoeyen et al. 1988, Science 239:1534-36). The recently described "gene conversion metagenesis" strategy for the production of humanized monoclonal antibody may also be employed in the production of humanized antibodies (Carter et al. 1992 Proc. Natl. Acad. Sci. U.S.A. 89:4285-89). Alternatively, techniques for generating the recombinant phase library of random combinations of heavy and light regions may be used to prepare recombinant antibodies (e.g. Huse et al. 1989 Science 246:1275-81).

Antibody fragments which contain the idiotype of the molecule such as Fu F(ab1) and F(ab2) may be generated by known techniques. For example, such fragments include but are not limited to: the F(ab) E2 fragment which can be produced by pepsin digestion of the intact antibody molecule; the Fab' fragments which can be generated by reducing the disulfide bridges of the F(ab')2 fragment, and the two Fab fragments which can be generated by treating the antibody molecule with papain and a reducing agent. Alternatively, Fab expression libraries may be constructed (Huse et al. 1989, Science 246:1275-1281) to allow rapid and easy identification of monoclonal Fab fragment with the desired specificity to a protein.

### Adjuvants

"Adjuvant" means a composition comprised of one or more substances that enhances the immunogenicity and efficacy of a vaccine composition. Non-limiting examples of suitable adjuvants include squalane and squalene (or other oils of animal origin); block copolymers; detergents such as Tween®-80; Quil® A, mineral oils such as Drakeol or Marcol, vegetable oils such as peanut oil; Corynebacterium-derived adjuvants such as Corynebacterium parvum; Propionibacterium-derived adjuvants such as Propionibacterium acne; Mycobacterium bovis (Bacillus Calmetic and Guerinn or BCG); interleukins such as interleukin 2 and interleukin-12; monokines such as interleukin 1; tumour necrosis factor; interferons such as gamma interferon; combinations such as saponin-aluminium hydroxide or Quil-A aluminium hydroxide; liposomes; ISCOM adjuvant; mycobacterial cell wall extract; synthetic glycopeptides such as muramyl dipeptides or other derivatives; Avridine; Lipid A; dextran sulfate; DEAE-Dextran or DHAE-Dextran with aluminium phosphate; carboxypolymethylene such as Carbopol' EMA; acrylic copolymer emulsions such as Neocryl A640 (e.g. U.S. Pat. No. 5,047,238); vaccinia or animal posvirus proteins; sub-viral particle adjuvants such as cholera toxin, or mixtures thereof.

As used herein, stringent conditions are those that (1) employ low ionic strength and high temperature for washing, for example, 0.015 M NaCl/0.0015 M sodium citrate/0.1 % NaDodSO4 at 50°C; (2) employ during hybridisation a denaturing agent such as formamide, for example, 50% (vol/vol) formamide with 0.1% bovine serum albumin, 0.1% Ficoll, 0.1% polyvinylpyrrolidone, 50 mM sodium phosphate buffer at pH 6.5 with 750 mM NaCl, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS and 10% dextran sulfate at 42°C in 0.2 x SSC and 0.1% SDS

As will be understood the present invention includes within its scope DNA vaccination. Further information regarding DNA vaccination may be found in Donnelly et al, Journal of Immunological Methods 176(1994) 145-152, the disclosure of which is incorporated herein by reference.

Throughout this specification the word "comprise", or variations such as "comprises" or "comprising", will be understood to imply the inclusion of a stated element or integer or group of elements or integers but not the exclusion of any other element or integer, or group of elements or integers.

### Preparation of the P. gingivalis library for sequencing.

To determine the DNA sequence of *P. gingivalis* genomic DNA was isolated from *P. gingivalis* strain W50 (ATCC 53978) essentially by the method described by Mamur J. ( J. Mol. Biol. 3, 208-218, 1961). Cloning of DNA fragments was performed essentially as described by Fleischmann et al., (Science; 269, 496-512, 1995)(2). Briefly, purified genomic DNA from *P. gingivalis* was nebulized to fragment the DNA and was treated with Ba131 nuclease to create blunt ends then run twice through preparative 1% agarose gels. DNA fragments of 1.6-2.0 kb were excised from the gel and the DNA recovered. This DNA was then ligated to the vector pUC18 (*Sma*I digested and dephosphorylated; Pharmacia) and electrophoresed through a 1% preparative agarose gel. The fragment comprising linear vector plus one insert was excised, purified and this process repeated to reduce any vector without insert contamination. The recovered vector plus insert DNA was blunt-ended with T4 DNA polymerase, then a final ligation to produce circular DNA was performed. Aliquots of Epicurian Coli Electroporation-Competent Cells (Stratagene) were transformed with the ligated DNA and plated out on SOB agar antibiotic diffusion plates containing X-gal and incubated at 37°C overnight. Colonies with inserts appeared white and those without inserts (vector alone) appeared blue. Plates were stored at 4°C until the white clones were picked and expanded for the extraction of plasmid DNA for sequencing.

### DNA sequencing

Plasmid DNA was prepared by picking bacterial colonies into l.5ml of LB, TB or SOB broth supplemented with 50-100ug/ml Ampicillin in 96 deep well plates. Plasmid DNA was isolated using the QIAprep Spin or QIAprep 96 Turbo miniprep kits (QIAGEN GmbH, Germany). DNA was eluted into a 96 well gridded array and stored at -20C.

Sequencing reactions were performed using ABI PRISM Dye Terminator and ABI PRISM BIGDye Terminator Cycle Sequencing Ready Reaction kits with AmpliTaq DNA polymerase FS (PE Applied Biosystems, Foster City, CA) using the M13 Universal forward and reverse sequencing primers. Sequence reactions were conducted on either a Perkin-Elmer GeneAmp 9700 (PE Applied Biosystems) or Hybaid PCR Express (Hybaid, UK) thermal cyclers. Sequencing reactions were analysed on ABI PRISM 377 DNA sequencers (PE Applied Biosystems).

A number of the sequences obtained are set out below. The relationship between these sequences is set out in Table 1. The initiation codon was calculated using a combination of sequence homology alignment (FASTA), signal sequence prediction (PSORT, SignalP) or ORF prediction (GeneMark).

**Table 1: Reference table indicating the relationships of each sequence ID to the selected proteins.**

| **Protein name** | **DNA sequence of complete ORF** | **Amino acid sequence of complete ORF** | **DNA sequence of protein** | **Amino acid sequence of protein** |
|---|---|---|---|---|
| PG1 | 1 | 265 | 122 | 386 |
| PG3 | 45 | 309 | 170 | 434 |
| PG30 | 46 | 310 | 171 | 435 |
| PG75 | 95 | 359 | 229 | 493 |
| PG90 | 112 | 376 | 254,255 | 518,519 |
| PG96 | 118 | 382 | 261 | 525 |
| PG97 | 119 | 383 | 262 | 526 |
| PG99 | 121 | 385 | 264 | 528 |
| PG100 | 3 | 267 | 124 | 388 |
| PG104 | 6 | 270 | 127 | 391 |

### DNA sequence analysis

DNA files in FASTA format were converted to GCG format files and imported into a database. The DNA files were translated into amino acid files using the program Flip obtained from ANGIS(Australian Genomic Information Service, University of Sydney, Australia). A series of bioinformatic analyses were performed on the proteins in order to select potential vaccine candidates. The programs used were FASTA homology searching (1), PSORT (2,3), SignalP (4), TopPred (5), and GeneMark (6). The proteins and their bioinformatic results were stored in the custom written database for search and retrieval of proteins with the desired characteristics

The FASTA homology results for these proteins were then examined for any alignment with a protein suggesting surface location or vaccine efficacy. All proteins were searched for homology against a non-redundant bacterial protein database compiled by ANGIS using the FASTA algorithm. The settings used for the FASTA searches were Ktup = 2, gap creation penalty = -12, gap extension penalty = -2, width for deriving alignment in opt = 16 and the Blosum 50 scoring matrix. Individual FASTA search results were examined for significant homology by statistical probability and amino acid alignments. The results are set out in Table 2.

Protein files were then trimmed to the first, second, third, fourth and fifth methionine residues using a protein trimming program (ANGIS). The trimmed proteins were then subjected to PSORT analysis for the detection of signal sequences and the prediction of cell location. Proteins exhibiting a PSORT probability of outer membrane >0.8 were considered to indicate surface localisation. A second signal sequence detection program SignalP was also performed and, in certain instances, this program detected signals not identified with PSORT. All proteins identified by other methods were also analysed by PSORT and SignalP. Previously, the C-terminal amino acid of bacterial outer membrane proteins has been shown to be important for the assembly of the protein on the outer membrane (7). A typical structure definition for outer membrane proteins has been determined as the presence of a signal sequence at the N-terminus and a tyrosine or phenylalanine at the C-terminus. A number of the selected proteins exhibit this characteristic structure. The program TopPred was used to determine the presence and number of membrane spanning domains (MSDs) and the presence of such sequences indicates a preference to be attached to membranes such as the outer membrane. The results of PSORT, SignalP and TopPred analyses with the C-terminal amino acids of the selected proteins are set out in Table 3.

The 70 amino acids from the C-terminus of a number of *P. gingivalis* outer membrane proteins share 50-100% protein sequence identity. These proteins included RGP1, RGP2, KGP, HagA, HagC, HagD, prtH and prtT. This conserved motif may be involved in the attachment or sorting of proteins to the outer membrane. The protein data set was searched using FASTA homology as described above and the results for PG96 and PG97 are listed in Table 4

**Table 2: FASTA protein homology results of complete ORFs against a non-redundant protein database.**

| **Protein name** | **Homology description** | **Genbank accession number** | **Length of homolog** | **Length of *P. gingivalis* protein** | **FASTA homology results** | | |
|---|---|---|---|---|---|---|---|
| | | | | | **identity %** | **Overlap** | **E value** |
| **PG1** 48kD | outer membrane protein, Actinobacillus pleuropneumoniae | U24492 | 449aa | 451aa | 32 | 454aa | 1.40E-42 |
| **PG3** Outer | membrane porin F adhesin, Pseudomonas fluorescens | U19743 | 317aa | 223aa | 35 | 187aa | 1.10E-10 |
| **PG30** | Putative NlpD lipoprotein, Aquifex aeolicus | AE0007 54 | 187 aa | 337aa | 42 | 42aa | 1.80E-12 |
| **PG75** | Class 3 outer membrane porin (porB), Neisseria meningitidis | U0719 | 332aa | 391aa | 23 | 239aa | 4.60E-01 |

**Table 3: Results of PSORT, SignalP and TopPred analysis of the proteins. The signal present column indicates the presence of a signal sequence detected with either PSORT or SignalP. The terms in parentheses indicates the type of signal sequence as determined by PSORT. The cell location & probability values are generated by PSORT and represent the probability of the protein being in the cell compartments outer membrane (OM), inner membrane (IM), periplasmic space (PC) or cytoplasm (C). The number of transmembrane domains (TMDs) was determined by TopPred and does not include uncleavable signal sequences.**

| **Protein name** | **Protein seqID number** | **Protein Length** | **Signal Present** | **Methionine in ORF** | **Signal P cleavage site** | **PSORT cleavage site** | **Cell Location & probability** | | | | **C-terminal Amino Acid** | **Number of TMD's** |
|---|---|---|---|---|---|---|---|---|---|---|---|---|
| | | | | | | | **OM** | **IM** | **PS** | **C** | | |
| PG1 | 386 | 451aa | Y | 1 | 24 | 34 | 0 | 0 | 0 | 0.22 | N | 0 |
| PG3 | 434 | 223aa | Y (lipoprotein) | 1 | - | 18 | 0.79 | 0.76 | 0 | 0 | K | 3 |
| PG30 | 435 | 337aa | Y | 1 | 21 | 21 | 0.24 | 0 | 0.4 | 0 | K | 0 |
| PG75 | 493 | 391a | Y | 1 | 26 | 26 | 0.94 | 0 | 0.3 | 0 | H | 1 |
| PG96 | 525 | 563aa | Y | 1 | 23 | 23 | 0.40 | 0 | 0.33 | 0 | K | 0 |
| PG97 | 526 | 437aa | Y | 1 | 23 | 23 | 0.32 | 0 | 0.65 | 0 | Q | 0 |
| PG90 | 519 | 228aa | Y | 2 | 21 | 20 | 0.85 | 0 | 0.44 | 0 | K | 0 |
| PG99 | 528 | 461aa | Y (uncleavable | 1 | 22 | 20 | 0 | 0 | 0.3 | 0 | R | 0 |
| PG100 | 388 | 279aa | Y | 1 | 20 | 18 | 0.26 | 0 | 0.54 | 0 | I | 0 |
| PG104 | 391 | 391aa | Y | 1 | 17 | 17 | 0.62 | 0 | 0.22 | | R | 0 |

**Table 4: Percentage identity and percentage similarity of various proteins with the 70 amino acids from the C-terminal of the P. gingivalis arginine protease 1 (RGP1), arginine protease 2 (RGP2), and the cysteine protease/hemagglutinin (prtT).**

| **Protein name** | | **Percent identity** | | | | **Percent similarity** | | |
|---|---|---|---|---|---|---|---|---|
| | | **RGP1** | **RGP2** | **prtT** | | **RGP1** | **RGP2** | **prtT** |
| **PG96** | | 0 | 13 | 20 | | 0 | 24 | 43 |
| **PG97** | | 10 | 26 | 33 | | 14 | 47 | 61 |
| **PG99** | | 19 | 0 | 26 | | 41 | 0 | 54 |
| **PG100** | | 20 | 21 | 24 | | 39 | 57 | 41 |
| **PG104** | | 10 | 23 | 26 | | 46 | 44 | 49 |

### Cloning, expression and purification of recombinant P. gingivalis genes.

### PG1

Oligonucleotides to the 5' and 3' regions of the deduced protein were used to amplify the gene of interest from a preparation of *P. gingivalis* W50 genomic DNA using the TaqPlus Precision PCR System ( Stratagene) and a PTC-100 (MJ Research) thermal cycler or similar device. The 5' oligonucleotide primer sequence was GCGCCATATGCTGGCCGAACCGGCC, the 3' oligonucleotide primer sequence was GCGCCTCGAGTCAATTCATTTCCTTATAGAG. The PCR fragment was purified, digested with Nde I, Xho I restriction enzymes (Promega) and ligated into the corresponding sites of the plasmid pProEx-1 (Gibco-BRL) and transformed into *E. coli* ER1793 cells (a gift from Elizabeth Raleigh, New England Biolabs). A resulting clone expressing the correct insert was selected and induced with or without 0.1mM IPTG (Promega) for expression of the recombinant protein. Expression of the recombinant protein was determined by SDS-PAGE analysis and Western Blot using the one of the rabbit antisera described above or an anti-hexahistidine antibody (Clontech) that detects the hexahistidine tag that was fused to *the P. gingivalis* recombinant protein. PG1 was purified by disruption of the *E. coli* cells by sonication in binding buffer (Novagen) and solubilisation by the addition of sarkosyl (N-Lauroyl sarcosine) to a 1% final concentration. There after the preparation was diluted to 0.1% sarkosyl in binding buffer, bound to a Nickel-nitrilotriacetic acid column (Ni-NTA; Qiagen), after washing bound proteins were eluted with 1M imidazole in elution buffer (Novagen) according to the Qiagen recommendations with 0.1% sarkosyl added to all buffers. Following purification samples were dialysed against 500mM NaCl, 20mM Tris, 0.1% sarkosyl at pH7.4 to remove the imidazole, concentrated as required and stored at 4°C until used. Purity and antigenicity were assessed by SDS-PAGE and Western blot using selected antisera (from those described above) and the protein concentration was determined by the BCA assay (Pierce).

### PG3

The methods used for PG3 were essentially the same as for PG1 with the following exceptions. The 5' oligonucleotide primer sequence was
GCGCGTATACATGAAGAAATCAAGTGTAG, the 3' oligonucleotide primer sequence was GCGCAGATCTCTTCAGCGTACCTTGCTGTG and DNA was amplified with Pfu DNA polymerase (Stratagene). The PCR product was cloned directly into pCR-Blunt and transformed into E. *coli* Top l OF' (InVitrogen) before subcloning into the expression plasmid pGex-stop RBS(IV) using the Bst Z171 and Bgl II restriction sites and transformed into *E. coli* BL21DE3 (Pharmacia Biotech). The following modifications were made to the purification of PG3 from the PG1 method. Cells expressing the recombinant protein were disrupted by sonication in binding buffer and the insoluble inclusion bodies concentrated by centrifugation. Inclusion bodies were then solubilised in 6M urea (Sigma) in binding buffer and eluted with 6M urea added to the elution buffer. In some instances 6M guanidine hydrochloride (Sigma) was used instead of urea for these steps. Urea (or guanidine hydrochloride when it was substituted) was removed from the purified protein by sequential dialysis against reducing levels of urea (3M then 1.5M then 0.5M then 0M urea all in 50mM Tris, 500mM NaCl, 8% glycerol, pH7.4). Purified protein was stored frozen at -80°C until required. Protein concentration was determined by the Coomassie Plus protein assay (Pierce).

### PG30

The methods used for PG30 were essentially the same as for PG3 with the following exceptions. The predicted N-terminal signal sequence was removed from the recombinant protein. The 5' oligonucleotide primer sequence was
TACGGAATTCGTGACCCCCGTCAGAAATGTGCGC, the 3' oligonucleotide primer sequence was CTATGCGGCCGCTTTGATCCTCAAGGCTTTGCCCGG and DNA was amplified with the Tth XL PCR kit. The PCR product was cloned into the expression plasmid pET24a using the Eco RI and Not I restriction sites and transformed into *E. coli* BL21DE3. Expression studies and immunoreactivity studies were carried out on whole *E. coli* lysates of PG30. 10ml cultures of recombinant *E. coli* were grown to an OD of 2.0 (A₆₀₀ₙₘ ) in terrific broth and the cells were induced with 0.5mM IPTG and samples taken for analysis at 4 hours post induction. Purification was not done for these studies.

### PG75

The methods used for PG75 were essentially the same as for PG30 with the following exceptions. The predicted N-terminal signal sequence was removed from the recombinant protein. The 5' oligonucleotide primer sequence was
GGCGGGATCCGCTCAGGAGCAACTGAATGTGGTA, the 3' oligonucleotide primer sequence was GAGTGCGGCCGCTGTGGAACAAATTGCGCAATCCATC and DNA was amplified with the Tth XL PCR kit. The PCR product was cloned into the expression plasmid pET24a using the Bam HI and Not I restriction sites and transformed into *E. coli* BL21DE3. Expression studies and immunoreactivity studies were carried out on whole *E. coli* lysates. Purification was not done for these studies.

### PG90

The methods used for PG90 were essentially the same as for PG30 with the following exceptions. The predicted N-terminal signal sequence was removed from the recombinant protein. The 5' oligonucleotide primer sequence was
GGCAGAATTCCAAACAACGACGAACAGTAGCCGG, the 3' oligonucleotide primer sequence was GAGTGCGGCCGCTTTTTTGTTGTGATACTGTTTGGGC and DNA was amplified with the Tth XL PCR kit. The PCR product was cloned into the expression plasmid pET24a using the Eco RI and Not I restriction sites and transformed into *E. coli* BL21DE3. Expression studies and immunoreactivity studies were carried out on whole *E. coli* lysates. Purification was not done for these studies.

### PG96

The methods used for PG96 were essentially the same as for PG30 with the following exceptions. The predicted N-terminal signal sequence was removed from the recombinant protein. The 5' oligonucleotide primer sequence was
TGCTGAGCTCCAAACGCAAATGCAAGCAGACCGA, the 3' oligonucleotide primer sequence was GAGTGCGGCCGCTTTTGAGAATTTTCATTGTCTCACG and DNA was amplified with the Tth XL PCR kit. The PCR product was cloned into the expression plasmid pET24a using the Sac I and Not I restriction sites and transformed into *E. coli* BL21DE3. Expression studies and immunoreactivity studies were carried out on whole *E. coli* lysates. Purification was not done for these studies.

### PG97

The methods used for PG97 were essentially the same as for PG30 with the following exceptions. The predicted N-terminal signal sequence was removed from the recombinant protein. The 5' oligonucleotide primer sequence was
GGCGGGATCCCAGTTTGTTCCGGCTCCCACCACA, the 3' oligonucleotide primer sequence was GAGTGCGGCCGCTCTGTTTGATGAGCTTAGTGGTATA and DNA was amplified with the Tth XL PCR kit. The PCR product was cloned into the expression plasmid pET24a using the Bam HI and Not I restriction sites and transformed into *E. coli* BL21DE3. Expression studies and immunoreactivity studies were carried out on whole *E. coli* lysates. Purification was not done for these studies.

### PG99

The methods used for PG99 were essentially the same as for PG30 with the following exceptions. The predicted N-terminal signal sequence was removed from the recombinant protein. The 5' oligonucleotide primer sequence was
TGCTGAATTCAAGGACAATTCTTCTTACAAACCT, the 3' oligonucleotide primer sequence was GAGTGCGGCCGCTTCGAATCACGACTTTTCTCACAAA and DNA was amplified with the Tth XL PCR kit. The PCR product was cloned into the expression plasmid pET24a using the Eco RI and Not I restriction sites and transformed into *E. coli* BL21DE3. Expression studies and immunoreactivity studies were carried out on whole *E. coli* lysates. Purification was not done for these studies.

### PG100

The methods used for PG100 were essentially the same as for PG30 with the following exceptions. The predicted N-terminal signal sequence was removed from the recombinant protein. The 5' oligonucleotide primer sequence was
GGCAGAATTCCAGTCTTTGAGCACAATCAAAGTA, the 3' oligonucleotide primer sequence was GAGTGCGGCCGCTGATAGCCAGCTTGATGCTCTTAGC and DNA was amplified with the Tth XL PCR kit. The PCR product was cloned into the expression plasmid pET24a using the Eco RI and Not I restriction sites and transformed into *E. coli* BL21DE3. Expression studies and immunoreactivity studies were carried out on whole *E. coli* lysates. Purification was not done for these studies.

### PG104

The methods used for PG104 were essentially the same as for PG30 with the following exceptions. The predicted N-terminal signal sequence was removed from the recombinant protein. The 5' oligonucleotide primer sequence was
GAACGGATCCAACGTGTCTGCTCAGTCACCCCGA, the 3' oligonucleotide primer sequence was GAGTGCGGCCGCTTCTGAGCGATACTTTTGCACGTAT and DNA was amplified with the Tth XL PCR kit. The PCR product was cloned into the expression plasmid pET24a using the Bam HI and Not I restriction sites and transformed into *E. coli* BL21DE3. Expression studies and immunoreactivity studies were carried out on whole *E. coli* lysates. Purification was not done for these studies.

### Animal antisera and human patient sera.

Various antisera were raised for detecting the expression and refolding of the recombinant *P. gingivalis* proteins. A whole cell antisera was raised by injecting New Zealand White rabbits with 3 doses of sonicated *P. gingivalis* (strain W50) containing approximately 2mg of protein. The first dose was given in Freunds complete adjuvant (FCA) and the second and third doses were given in Freunds incomplete adjuvant (IFA) at 3 week intervals. Doses (1ml) were given intramuscularly into the hind legs and rabbits bled 7 days after the last dose, the blood clotted and serum removed and stored at -20°C until required. A second rabbit antisera was produced in a similar manner but using a sarkosyl insoluble fraction (each dose was 0.69mg of protein) derived from *P. gingivalis* W50 according to the method of Doidg and Trust T. *et al* 1994 as the immunogen. A third rabbit antisera was produced in a similar manner to the first only the sarkosyl soluble fraction (1mg of protein per dose) derived from *P. gingivalis* W50 cells according to the method of Doidg P. and Trust TJ. (1994 Infect Immun 62:4526-33) was used as the immunogen.

A "protected rat serum" pool was also used in these studies and was obtained from rats immunised with formalin killed whole *P. gingivalis* cells in FIA (strain ATCC 33277; 2 doses of 2x10⁹ cells, 3 weeks apart). Rats were then challenged 2 weeks after their last dose with live *P. gingivalis* cells (strain 33277) given orally as previously described (Klaussen B. et al. 1991, Oral Microbiol Immunol 6:193-201) and the serum obtained from these rats 6 weeks after the final challenge inoculation at the time of sacrifice.

Human sera were obtained from adult patients undergoing treatment or assessment for periodontitis at an outpatient clinic. These patients had at least 6 teeth with 6mm attachment loss and had *P. gingivalis* present in their sub-gingival plaque as detected using a *P. gingivalis* specific DNA probe. Sera was pooled from these patients and compared to a pool of sera from periodontally healthy patients.

### Immunization and Murine Lesion Model Protocols

The mouse abscess model was used to assess the efficacy of immunising mice with recombinant *P. gingivalis* proteins in protecting mice from formation of a subcutaneous abscess. This model has been used by others as a predictor of potential vaccines against periodontal disease (Bird PS, et al. 1995 J. Periodontol. 66:351-362. BALB/c mice 6-8 weeks old were immunised by subcutaneously injecting them with 0.1 ml containing either 10 or 20µg of recombinant *P. gingivalis* protein, 20µg of *E. coli* lysate protein, 2 x 10⁹ formalin killed cells of *P. gingivalis* strain 33277 emulsified in incomplete Freund's adjuvant (IFA; Sigma) on day 0. At day 21 mice were re-injected with the same dose and then bled 1 week later and evaluated for antibody levels. At day 35 mice all mice were challenged with approximately 2 x 10⁹ cells of live *P. gingivalis* (ATCC 33277) by subcutaneous injection in the abdomen. Following challenge mice were monitored daily for weight loss and the size of the lesion measured for the next 10 days. Lesion sizes were measured by length and width and expressed as mm². Groups were statistically analysed using a Kruskal-Wallis one-way ANOVA and were also individually examined using the unpaired t test or Mann-Whitney rank sum test using the Instat statistical package.

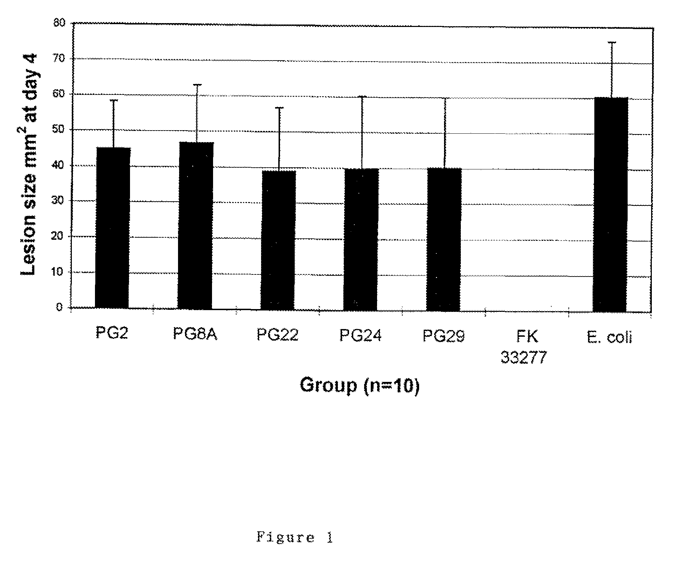

Figure 1 shows the results of one experiment at day 4 after challenge (lesions were at maximum size at this time point). Control mice immunised with *E. coli* lysate showed large lesions while mice immunised with killed cells of *P. gingivalis* strain 33277 were fully protected. This indicates that whole cells provide protection against *P. gingivalis* while *E. coli* protein immunised mice were not protected. Mice given the various PG recombinant proteins showed significant levels of protection for PG2, PG22, PG24 and PG29 (p<0.05 unpaired t test) while PG8A was not quite significantly different (p=0.07) compared to the *E. coli* control group.

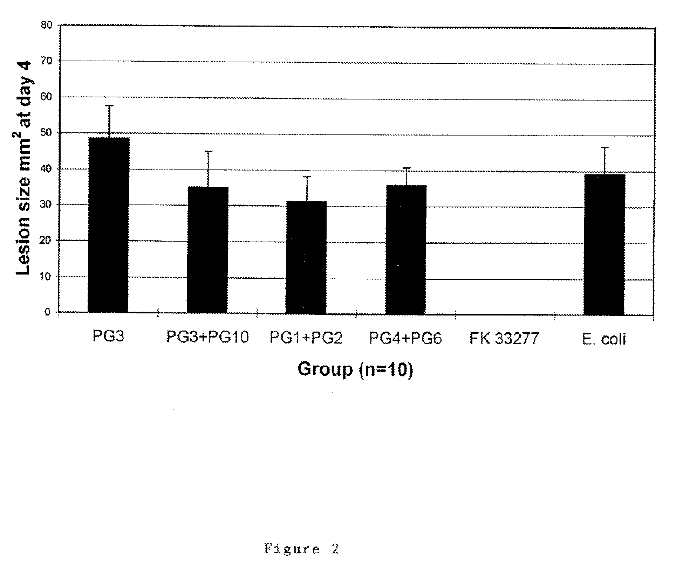

Figure 2 shows the results of a separate experiment using combinations of recombinant proteins. Mice given PG1 + PG2 showed a significant level of protection compared to control mice give *E. coli* lysate (p<0.026 unpaired t test).

### Immunoscreening

Cloned candidates were cultured in 15ml of Terrific broth, induced with IPTG and sampled at 4h post-induction. One ml of culture was removed, pelleted and the cells resuspended in a volume of PBS determined by dividing the OD A₆₀₀ₙₘ of the culture by 8. An aliquot of lysate (100µl) was added to 100µl of 2x sample reducing buffer (125mM Tris pH 6.8, 20% glycerol, 4% SDS, 80mM DTT, 0.03% bromophenol blue) and boiled for 10min. SDS-PAGE was performed according to the method of Laemmli UK. 1970 (Nature 227:680-685) using 4-20% 1.0mm Tris-Glycine gels (Novex) according to the manufacturers recommendations. Proteins were transferred onto Hybond-C Extra nitrocellulose membranes (Amersham) by transblotting and the membranes were then blocked for 2h at room temperature (RT) in 5% skim milk in 20mM Tris, 0.5M NaCl, 0.05% Tween-20, pH 7.5 (TTBS).

Immunoscreening was performed separately with the rabbit anti-*P. gingivalis* whole cell serum, the rat protective serum, a pool of human periodontal patients serum, and in many cases an anti-T7-Tag antibody HRP conjugate (Novagen). Prior to use, the rabbit, rat and human sera were diluted 1/5000, 1/1000 and 1/500 respectively in 5% skim milk in TTBS and absorbed with 100µl (for the rabbit serum) or 250µl (for the rat and human sera) *E. coli* extract (20mg/ml; Promega) for 6h at RT.

Membranes were incubated overnight at RT with the absorbed antisera, or for 1 hr at RT with 1/5000 diluted anti-T7-Tag conjugate. Following 3x10min washes with TTBS, HRP-conjugated anti-rabbit (Silenus), anti-mouse (Silenus) or anti-human (KPL) antibody, diluted 1/5000 in 5% skim milk in TTBS, was added for 1h at RT. Membranes were washed as before, prior to addition of TMB membrane peroxidase substrate (KPL) for detection of immunoreactive proteins. Results of reactivity for the recombinant *P. gingivalis* proteins is shown in Table 5.

**Table 5: Immunoblot results of proteins expressed in E. coli against rabbit, rat and human antisera. Deduced MW was calculated from amino acid sequence of the P. gingivalis proteins, some of which had their N-terminal signal sequences removed. Apparent MW was determined from SDS-PAGE gels. The N- and C-terminal tags add approximately 2.5 KDa to the deduced MW of the recombinant proteins. The symbols are + positive, - negative, +/- weak positive, ND not done.**

| **Protein number** | **Deduced MW (KDa)** | **Apparent MW (KDa)** | **Antisera reactivity** | | | |
|---|---|---|---|---|---|---|
| | | | **T7** | **Rabbit** | **Rat** | **Human** |
| PG1 | 47.5 | 63 | ND | - | - | - |
| PG3 | 22.6 | 18.3 | ND | -**^{a}** | - | - |
| PG30 | 35.1 | 46.9 | + | - | - | - |
| PG75 | 40.7 | 46.7 | + | - | - | - |
| PG90 | 23 | 28.4 | + | - | - | - |
| PG96 | 59.3 | 70.3 | + | + | + | + |
| PG97 | 44.4 | 57.5 | + | - | + | + |
| PG99 | 40.7 | 55.6 | + | - | + | + |
| PG100 | 29.6 | 10.8 | + | - | - | - |
| PG104 | 40.7 | 57.5 | + | - | - | + |

| | | | | | | |
|---|---|---|---|---|---|---|
| **a.** Positive reaction detected with the rabbit antiserum to sarkosyl insoluble *P. gingivalis* antigen. | | | | | | |

It will be appreciated by persons skilled in the art that numerous variations and/or modifications may be made to the invention as shown in the specific embodiments without departing from the spirit or scope of the invention as broadly described. The present embodiments are, therefore, to be considered in all respects as illustrative and not restrictive.

### References.

1. Lipman DJ, Pearson WR. 1985. Rapid and sensitive protein similarity searches. Science 277:1435-1441.
2. Horton, P. and Nakai, K. (1996). A probabilistic classification system for predicting the cellular localization sites of proteins. Intellig. Syst. Mol. Biol. 4: 109-115.
3. Nakai K, Kanehisa M. 1991. Expert systems for predicting protein localization sites in Gram-negative bacteria. Proteins: Structure, Function, and Genetics 11:95-110.
4. Nielsen H, Engelbrecht J, Brunak S and von Heijne G. 1997. Identification of prokaryotic and eukaryotic signal peptides and prediction of their cleavage sites. Protein Engineering 10, 1-6.
5. Claros MG and G von Heijne. (1994). TopPred II: an improved software for membrane protein structure predictions. Comput. Appl. Biosci. 10: 685-686.
6. Borodovsky M, Rudd KE, and EV Koonin. (1994). Intrinsic and extrinsic approaches for detecting genes in a bacterial genome. Nucleic Acids Res. 22:4756-4767.
7. Struvye M, Moons M, Tommassen J. 1991. Carboxy-terminal phenylalanine is essential for the correct assembly of a bacterial outer membrane protein J. Mol. Biol. 218:141-148.
8. Aduse-Opoku J, Slaney JM, Rangarajan M, Muir J, Young KA, Curtis MA. 1997. The Tla receptor protein of Porphyromonas gingivalis W50: a homolog of the RI precursor (PrpRI) is an outer membrane receptor required for growth on low levels of hemin. J. Bacteriol. 179:4778-4788.
9. Needleman SB, Munsch CD. 1970. Ageneral method applicable to the search of similarity in the amino acid sequence of two proteins. J. Molec. Biol. 48: 443-453.

Tyr Phe Gly Leu Leu Asp Asp Gln Pro Ala Ser Val Asn Ile Gly Trp
    530                 535                 540
Arg Asn Tyr Asn Met Ala Val Gly Arg Phe Pro Val Arg Thr Pro Ala
545                 550                 555                 560
Glu Ala Arg Ile Ala Val Asp Lys Thr Ile Arg Tyr Glu Glu Asp Arg
                565                 570                 575
Glu Ser Gly Ala Trp Arg Ile Arg Ala Cys Phe Ala Ala Asp Asn Gly
            580                 585                 590
Asp Lys His Ala Thr Glu Thr Ser Arg Leu Ile Asp Thr Val Lys Arg
        595                 600                 605
Tyr Ala Pro Ala Ile Met Pro Val Arg Ala Phe Gln Asp Val Tyr Pro
    610                 615                 620
His Val Ile Glu Asn Gly Leu His Ser Ile Pro Gly Ala Lys Lys Lys
625                 630                 635                 640
Met Leu Glu Thr Leu Gln Ser Gly Ile Ile Leu Leu Asn Tyr Ala Gly
                645                 650                 655
His Gly Gly Pro Ala Gly Trp Ser Asp Glu His Leu Leu Thr Leu Asn
            660                 665                 670
Asp Ile His Lys Phe Asn Tyr Lys His Met Pro Ile Trp Ile Thr Ala
        675                 680                 685
Thr Cys Asp Phe Ala Asn Tyr Asp Ser Gln Thr Thr Ser Ala Gly Glu
    690                 695                 700
Glu Val Phe Leu His Glu Lys Ser Gly Thr Pro Ile Met Phe Ser Thr
705                 710                 715                 720
Thr Arg Val Val Tyr Asn Thr Gln Asn Glu Lys Ile Asn Gly Phe Met
                725                 730                 735
Leu Arg Arg Met Phe Glu Lys Ala Lys Asp Gly Arg Tyr Arg Thr Met
            740                 745                 750
Gly Glu Ile Ile Arg Ser Ala Lys Gln Gly Met Leu Ser Thr Val Phe
        755                 760                 765
Pro Asp Ser Ile Asn Gln Leu Ser Phe Phe Leu Met Gly Asp Pro Ser
    770                 775                 780
Val Arg Met Asn Leu Pro Thr His Lys Val Gln Leu Thr Ala Ile Asn
785                 790                 795                 800
Gly Gln Asp Pro Glu Gly Gln Tyr Gly Thr Ile Met Leu Lys Ser Leu
                805                 810                 815
Glu Arg Val Ala Leu Lys Gly Lys Val Thr Asp Glu Lys Gly Thr Phe
            820                 825                 830
Asp Glu Thr Phe Ser Gly Lys Val Phe Leu Thr Val Phe Asp Gly Arg
        835                 840                 845
Lys Lys Met Thr Ala Leu Glu Glu Glu Gly Asn Asp Leu Ser Leu Val
    850                 855                 860
Tyr Tyr Asp Tyr Pro Asn Val Met Tyr Ala Gly Ile Ala Glu Val Lys
865                 870                 875                 880
Asp Gly Leu Phe Glu Thr Ser Phe Ile Val Pro Lys Asp Val Asn Tyr
                885                 890                 895
Ser Glu His Glu Gly Arg Ile Asn Leu Tyr Ala Tyr Asn Glu Ser Thr
            900                 905                 910
Lys Ala Glu Ala Met Gly Val Asp Phe Ser Ile Arg Val Gln Pro Gly
        915                 920                 925
Ile Pro Asp Glu Val Thr Glu Asp Asn Thr Pro Pro Glu Ile Ile Ser
    930                 935                 940
Cys Phe Leu Asn Asp Ser Thr Phe Arg Ser Gly Asp Glu Val Asn Pro
945                 950                 955                 960
Thr Pro Leu Phe Met Ala Glu Val Phe Asp Leu Asn Gly Ile Asn Ile
                965                 970                 975
Thr Gly Ser Gly Val Gly His Asp Ile Thr Leu Cys Ile Asp Gly Arg
            980                 985                 990
Ala Asp Leu Thr Tyr Asn Leu Asn Ala Tyr Phe Thr Ser Ser Ala Thr
        995                 1000                1005
Asp Ala Gly Val Gly Thr Ile Leu Phe Met Ile Pro Ala Leu Ala Glu
    1010                1015                1020
Gly Asp His Thr Ala Arg Leu Thr Val Trp Asp Ile Phe Asn Asn Ala
1025                1030                1035                1040
Val His His Asp Phe Ser Phe Arg Val Val Asp Gly Ile Ala Pro Asp
                1045                1050                1055
Val Ala Asp Val Ile Leu Phe Pro Asn Pro Val Arg Glu Ser Ala Thr
            1060                1065                1070
Phe Arg Ile Phe His Asn Arg Pro Gly Ser Asp Leu Asn Val Ala Val
        1075                1080                1085
Glu Ile Tyr Asp Phe Thr Gly Arg Leu Val Asn Ser Leu Pro Val Lys
    1090                1095                1100
Thr Tyr Ser Ser Ser Tyr Gly Glu Pro Ile Glu Ile Lys Trp Asp Leu
1105                1110                1115                1120
Thr Ser Lys Tyr Gly Val Lys Ile Gly Asn Gly Phe Tyr Leu Tyr Arg
                1125                1130                1135
Cys Val Val Asn Ser Pro Gly Gly Gln Thr Ala Ser Met Ala Lys Lys
            1140                1145                1150
Met Ile Val Val Gly Gln
        1155

(2) INFORMATION FOR SEQ ID NO:524

     (i) SEQUENCE CHARACTERISTICS:
          (A) LENGTH: 961 amino acids
          (B) TYPE: amino acid

(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...961

(xi) SEQUENCE DESCRIPTION: SEQ ID NO:524

Met Lys Lys Leu Phe Pro Leu Leu Leu Leu Ile Leu Ser Ile Leu Val
1               5                   10                  15
Gly Cys Gly Lys Lys Glu Lys His Ser Val Thr Glu Ile Ala Arg Glu
            20                  25                  30
Lys Lys Arg Ile Thr Ala Leu Leu Tyr Glu Lys Glu Leu Pro Thr Asp
        35                  40                  45
Ser Val Lys Gln Leu Tyr Glu Asn Ser Val Gln Asn Lys Asn Leu Val
    50                  55                  60
Gly Gln Met Leu Phe Ala Ile Glu Val Gly Lys Arg Met Arg Asn Met
65                  70                  75                  80
Ser Gln Tyr Thr Asp Ala Met Leu Tyr His Gln Lys Gly Leu Asn Ala
                85                  90                  95
Ala Leu Asn Leu Arg Asp Thr Ile Val Ala Ala Gln Ala Trp Asn His
            100                 105                 110
Leu Gly Thr Asp Ser Arg Arg Ile Gly Ala Leu Ala Glu Ala Ser Asp
        115                 120                 125
Tyr His Tyr Lys Ala Leu Ser Leu Ile Glu Ser Phe Ser Gly Asn Gln
    130                 135                 140
Asn Arg Pro Ala Ile Lys Ala Arg Ser Ala Ala Leu Asn Gly Ile Gly
145                 150                 155                 160
Asn Ile Asn Leu Glu Leu Gly Tyr His Asp Glu Ala Glu Lys Asn Phe
                165                 170                 175
Leu Lys Ala Leu Gln Gly Glu Lys Glu Leu Asp Ser Pro Leu Gly Gln
            180                 185                 190
Ala Ile Asn Tyr Ala Asn Leu Gly Arg Ile Tyr Arg Gln Arg Lys Glu
        195                 200                 205
Tyr Asp Lys Ala Arg Thr Tyr Phe Leu Leu Ser Leu Glu Gln Asn Asn
    210                 215                 220
Met Ala Glu Asn Leu Met Gly Ile Gly Leu Cys Ser Ile Asn Leu Gly
225                 230                 235                 240
Glu Val Asp Glu Glu Lys Gly Asp Tyr Gln Lys Ala Leu Gln Glu Tyr
                245                 250                 255
Ala Thr Ala Tyr Lys Leu Met Glu Gln Leu Ser Asp Arg Trp His Trp
            260                 265                 270
Leu Asn Ser Cys Ile Pro Met Ala Arg Ile Asn Leu Lys Gln Gly Asn
        275                 280                 285
Glu Arg Leu Tyr Gln His Phe Ile Ser Leu Ala Glu Gly Thr Ala Lys
    290                 295                 300
Glu Ile Asn Ser Thr Ser His Leu Ile Glu Ile Tyr Asn Leu Gln Tyr
305                 310                 315                 320
Glu Asn Leu Glu Arg Lys Lys Glu Tyr Lys Gln Ala Leu Glu Ala Phe
                325                 330                 335
Cys Leu Ser Lys Thr Leu Ser Asp Ser Met Ser Ile Ala His Lys Val
            340                 345                 350
Ser Ser Ile Gln Glu Thr Arg Phe Asn Tyr Glu Arg Asn Lys Ser Gln
        355                 360                 365
Lys Glu Leu Glu Glu Ile Gln Gln Val Ser Lys Ala Lys Gln Glu Lys
    370                 375                 380
Ser Lys Phe Ile Leu Leu Ser Thr Leu Phe Ala Leu Phe Ile Ser Ile
385                 390                 395                 400
Leu Leu Ile Ser Val Leu Thr Tyr Ala Tyr Arg Gln Gly Lys Lys His
                405                 410                 415
Asn Lys Leu Ile Lys Glu Thr Asp Lys Leu Arg Ser Gly Phe Phe Thr
            420                 425                 430
Gly Ile Thr His Glu Phe Arg Thr Pro Ile Thr Val Ile Gln Gly Leu
        435                 440                 445
Asn Glu Lys Met Ser Ser Ser Pro Asp Leu Gln Ala Ser Asp Arg Thr
    450                 455                 460
Glu Leu His Lys Ile Ile Asp Arg Gln Ser Ser His Met Leu Asn Leu
465                 470                 475                 480
Val Asn Gln Leu Leu Asp Ile Cys Lys Ile Arg Ser Gly Val Ser Thr
                485                 490                 495
Pro Glu Trp Arg Asn Gly Asp Ile Val Ser Phe Val Gln Ile Leu Ile
            500                 505                 510
Asp Ser Phe Ala Pro Tyr Ala Gln Ala Gln Asp Ile Thr Leu Glu Leu
        515                 520                 525
Gln Pro Glu Ser Lys Pro Ile Val Val Asp Phe Val Pro Ser Tyr Leu
    530                 535                 540
Gln Lys Ile Ile Ser Asn Leu Leu Ser Asn Ala Ile Lys Tyr Ser Leu
545                 550                 555                 560
Ala Gly Gly Arg Val Val Ile Ser Leu Ala Lys Thr Lys Asn Glu Lys

565                 570                 575
Asn Leu Ile Ile Arg Val Ala Asp Asn Gly Ile Gly Ile Asp Lys Thr
            580                 585                 590
Asp Gln Ala His Ile Phe Asp Ile Phe Tyr Arg Gly Gln Ser Ala Thr
        595                 600                 605
Glu Lys His Gly Ser Gly Val Gly Leu Ser Phe Thr Asn Ile Leu Val
    610                 615                 620
Glu Asn Leu Arg Gly Thr Ile Lys Val Glu Ser Gln Pro Gly Lys Gly
625                 630                 635                 640
Ser Ala Phe Thr Ile Ser Ile Pro Thr Gln Asn Gln Ser Ser Ser Ala
                645                 650                 655
Glu Ile Leu Pro Trp Leu Pro Ser Ser Asp Asp Ile Val Met Pro Val
            660                 665                 670
His Ile Ala Pro Asp Asp Ser Pro Thr Ser Pro Met Val Ala Ala Leu
        675                 680                 685
Asn His Arg Phe Glu Asp Glu Arg Pro Thr Ile Leu Leu Val Glu Asp
    690                 695                 700
Asn Lys Asp Ile Asn Leu Leu Val Lys Leu Leu Leu Cys Asp Arg Tyr
705                 710                 715                 720
Asn Val Leu Ser Ala Ala Asn Gly Lys Glu Gly Ile Ala Leu Ala Thr
                725                 730                 735
Glu His Ile Pro Asp Ile Ile Ile Thr Asp Ile Met Met Pro Ile Met
            740                 745                 750
Asp Gly Ile Glu Met Thr Ile Arg Met Lys Gln Ser Pro Leu Leu Cys
        755                 760                 765
His Ile Pro Ile Val Ala Leu Thr Ala Lys Ser Thr Glu Gln Asp Arg
    770                 775                 780
Leu Glu Gly Ile Lys Ser Gly Val Val Ser Tyr Leu Cys Lys Pro Phe
785                 790                 795                 800
Ser Pro Glu Glu Leu Leu Met Arg Ile Glu Gln Leu Leu Lys Asp Arg
                805                 810                 815
Glu Leu Leu Lys Lys Phe Tyr Met Gln Lys Leu Met Leu Asp Arg Lys
            820                 825                 830
Pro Glu Glu Glu Pro Gln Pro Ile Asp Asp Ser Ser Met Gln Phe Leu
        835                 840                 845
Leu Ala Ala Lys Asp Ala Val Ser Gly Gly Ile Lys Gln Asn Pro Asp
    850                 855                 860
Phe Ser Ala Gln Asp Leu Ala Glu Lys Met Cys Met Ser Pro Ser Gln
865                 870                 875                 880
Leu Asn Arg Lys Leu Thr Ser Val Val Gly Cys Ser Thr Ile Gly Tyr
                885                 890                 895
Ile Gln Gln Ile Lys Ile Lys Leu Ala Cys Lys Leu Leu Ala Asp Glu
            900                 905                 910
Ser Lys Asn Ile Ser Asp Ile Ser Ile Glu Ala Gly Phe Ser Asp Pro
        915                 920                 925
Ala Tyr Phe Ser Arg Thr Phe Lys Arg Tyr Met Asn Cys Ser Pro Ser
    930                 935                 940
Gln Tyr Arg Gln Lys Leu Leu Ala Met Pro Gly Ser Asp Lys Glu Thr
945                 950                 955                 960
Val


(2) INFORMATION FOR SEQ ID NO:525

     (i) SEQUENCE CHARACTERISTICS:
          (A) LENGTH: 556 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...556

    (xi) SEQUENCE DESCRIPTION: SEQ ID NO:525

Met Lys Lys Leu Leu Gln Ala Lys Ala Leu Ile Leu Ala Leu Gly Leu
1               5                   10                  15
Phe Gln Leu Pro Ala Ile Ala Gln Thr Gln Met Gln Ala Asp Arg Thr
            20                  25                  30
Asn Gly Gln Phe Ala Thr Glu Glu Met Gln Arg Ala Phe Gln Glu Thr
        35                  40                  45
Asn Pro Pro Ala Gly Pro Val Arg Ala Ile Ala Glu Tyr Glu Arg Ser
    50                  55                  60
Ala Ala Val Leu Val Arg Tyr Pro Phe Gly Ile Pro Met Glu Leu Ile
65                  70                  75                  80
Lys Glu Leu Ala Lys Asn Asp Lys Val Ile Thr Ile Val Ala Ser Glu
                85                  90                  95
Ser Gln Lys Asn Thr Val Ile Thr Gln Tyr Thr Gln Ser Gly Val Asn
            100                 105                 110

Leu Ser Asn Cys Asp Phe Ile Ile Ala Lys Thr Asp Ser Tyr Trp Thr
        115                 120                 125
Arg Asp Tyr Thr Gly Trp Phe Ala Met Tyr Asp Thr Asn Lys Val Gly
    130                 135                 140
Leu Val Asp Phe Ile Tyr Asn Arg Pro Arg Pro Asn Asp Asp Glu Phe
145                 150                 155                 160
Pro Lys Tyr Glu Ala Gln Tyr Leu Gly Ile Glu Met Phe Gly Met Lys
                165                 170                 175
Leu Lys Gln Thr Gly Gly Asn Tyr Met Thr Asp Gly Tyr Gly Ser Ala
            180                 185                 190
Val Gln Ser His Ile Ala Tyr Thr Glu Asn Ser Ser Leu Ser Gln Ala
        195                 200                 205
Gln Val Asn Gln Lys Met Lys Asp Tyr Leu Gly Ile Thr His His Asp
    210                 215                 220
Val Val Gln Asp Pro Asn Gly Glu Tyr Ile Asn His Val Asp Cys Trp
225                 230                 235                 240
Gly Lys Tyr Leu Ala Pro Asn Lys Ile Leu Ile Arg Lys Val Pro Asp
                245                 250                 255
Asn His Pro Gln His Gln Ala Leu Glu Asp Met Ala Ala Tyr Phe Ala
            260                 265                 270
Ala Gln Thr Cys Ala Trp Gly Thr Lys Tyr Glu Val Tyr Arg Ala Leu
        275                 280                 285
Ala Thr Asn Glu Gln Pro Tyr Thr Asn Ser Leu Ile Leu Asn Asn Arg
    290                 295                 300
Val Phe Val Pro Val Asn Gly Pro Ala Ser Val Asp Asn Asp Ala Leu
305                 310                 315                 320
Asn Val Tyr Lys Thr Ala Met Pro Gly Tyr Glu Ile Ile Gly Val Lys
                325                 330                 335
Gly Ala Ser Gly Thr Pro Trp Leu Gly Thr Asp Ala Leu His Cys Arg
            340                 345                 350
Thr His Glu Val Ala Asp Lys Gly Tyr Leu Tyr Ile Lys His Tyr Pro
        355                 360                 365
Ile Leu Gly Glu Gln Ala Gly Pro Asp Tyr Lys Ile Glu Ala Asp Val
    370                 375                 380
Val Ser Cys Ala Asn Ala Thr Ile Ser Pro Val Gln Cys Tyr Tyr Arg
385                 390                 395                 400
Ile Asn Gly Ser Gly Ser Phe Lys Ala Ala Asp Met Thr Met Glu Ser
                405                 410                 415
Thr Gly His Tyr Thr Tyr Ser Phe Thr Gly Leu Asn Lys Asn Asp Lys
            420                 425                 430
Val Glu Tyr Tyr Ile Ser Ala Ala Asp Asn Ser Gly Arg Lys Glu Thr
        435                 440                 445
Tyr Pro Phe Ile Gly Glu Pro Asp Pro Phe Lys Phe Thr Cys Met Asn
    450                 455                 460
Glu Thr Asn Thr Cys Thr Val Thr Gly Ala Ala Lys Ala Leu Arg Ala
465                 470                 475                 480
Trp Phe Asn Ala Gly Arg Ser Glu Leu Ala Val Ser Val Ser Leu Asn
                485                 490                 495
Ile Ala Gly Thr Tyr Arg Ile Lys Leu Tyr Asn Thr Ala Gly Glu Glu
            500                 505                 510
Val Ala Ala Met Thr Lys Glu Leu Val Ala Gly Thr Ser Val Phe Ser
        515                 520                 525
Met Asp Val Tyr Ser Gln Ala Pro Gly Thr Tyr Val Leu Val Val Glu
    530                 535                 540
Gly Asn Gly Ile Arg Glu Thr Met Lys Ile Leu Lys
545                 550                 555

(2) INFORMATION FOR SEQ ID NO:526

      (i) SEQUENCE CHARACTERISTICS:
           (A) LENGTH: 428 amino acids
           (B) TYPE: amino acid
           (D) TOPOLOGY: linear

     (ii) MOLECULE TYPE: protein

    (iii) HYPOTHETICAL: YES

     (vi) ORIGINAL SOURCE:
           (A) ORGANISM: Porphyromonas gingivalis

     (ix) FEATURE:
           (A) NAME/KEY: misc_feature
           (B) LOCATION 1...428

     (xi) SEQUENCE DESCRIPTION: SEQ ID NO:526

Met Lys Leu Ser Ser Lys Lys Ile Leu Ala Ile Ile Ala Leu Leu Thr
1               5                   10                  15
Met Gly His Ala Val Gln Ala Gln Phe Val Pro Ala Pro Thr Thr Gly
            20                  25                  30
Ile Arg Met Ser Val Thr Thr Thr Lys Ala Val Gly Glu Lys Ile Glu
        35                  40                  45
Leu Leu Val His Ser Ile Glu Lys Lys Gly Ile Trp Ile Asp Leu Asn
    50                  55                  60
Gly Asp Ala Thr Tyr Gln Gln Gly Glu Glu Ile Thr Val Phe Asp Glu

65                  70                  75                  80
Ala Tyr His Glu Tyr Thr Ile Gly Thr Gln Thr Leu Thr Ile Tyr Gly
                85                  90                  95
Asn Thr Thr Arg Leu Gly Cys Arg Ser Thr Gly Ala Thr Ala Val Asp
            100                 105                 110
Val Thr Lys Asn Pro Asn Leu Thr Tyr Leu Ala Cys Pro Lys Asn Asn
        115                 120                 125
Leu Lys Ser Leu Asp Leu Thr Gln Asn Pro Lys Leu Leu Arg Val Trp
    130                 135                 140
Cys Asp Ser Asn Glu Ile Glu Ser Leu Asp Leu Ser Gly Asn Pro Ala
145                 150                 155                 160
Leu Ile Ile Leu Gly Cys Asp Arg Asn Lys Leu Thr Glu Leu Lys Thr
                165                 170                 175
Asp Asn Asn Pro Lys Leu Ala Ser Leu Trp Cys Ser Asp Asn Asn Leu
            180                 185                 190
Thr Glu Leu Glu Leu Ser Ala Asn Pro Arg Leu Asn Asp Leu Trp Cys
        195                 200                 205
Phe Gly Asn Arg Ile Thr Lys Leu Asp Leu Ser Ala Asn Pro Leu Leu
    210                 215                 220
Val Thr Leu Trp Cys Ser Asp Asn Glu Leu Ser Thr Leu Asp Leu Ser
225                 230                 235                 240
Lys Asn Ser Asp Val Ala Tyr Leu Trp Cys Ser Ser Asn Lys Leu Thr
                245                 250                 255
Ser Leu Asn Leu Ser Gly Val Lys Gly Leu Ser Val Leu Val Cys His
            260                 265                 270
Ser Asn Gln Ile Ala Gly Glu Glu Met Thr Lys Val Val Asn Ala Leu
        275                 280                 285
Pro Thr Leu Ser Pro Gly Ala Gly Ala Gln Ser Lys Phe Val Val Val
    290                 295                 300
Asp Leu Lys Asp Thr Asp Glu Lys Asn Ile Cys Thr Val Lys Asp Val
305                 310                 315                 320
Glu Lys Ala Lys Ser Lys Asn Trp Arg Val Phe Asp Phe Asn Gly Asp
                325                 330                 335
Ser Asp Asn Met Leu Pro Tyr Glu Gly Ser Pro Thr Ser Asn Leu Ala
            340                 345                 350
Val Asp Ala Pro Thr Val Arg Ile Tyr Pro Asn Pro Val Gly Arg Tyr
        355                 360                 365
Ala Leu Val Glu Ile Pro Glu Ser Leu Leu Gly Gln Glu Ala Ala Leu
    370                 375                 380
Tyr Asp Met Asn Gly Val Lys Val Tyr Ser Phe Ala Val Glu Ser Leu
385                 390                 395                 400
Arg Gln Asn Ile Asp Leu Thr His Leu Pro Asp Gly Thr Tyr Phe Phe
                405                 410                 415
Arg Leu Asp Asn Tyr Thr Thr Lys Leu Ile Lys Gln
            420                 425

(2) INFORMATION FOR SEQ ID NO:527

     (i) SEQUENCE CHARACTERISTICS:
          (A) LENGTH: 310 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc feature
          (B) LOCATION 1...310

    (xi) SEQUENCE DESCRIPTION: SEQ ID NO:527

Met Arg Lys Thr Ile Ile Phe Cys Leu Leu Leu Ala Leu Phe Gly Cys
1               5                   10                  15
Ser Trp Ala Gln Glu Arg Val Asp Glu Lys Val Phe Ser Ala Gly Thr
            20                  25                  30
Ser Ile Phe Arg Gly Ile Leu Glu Lys Val Lys Ala Pro Leu Met Tyr
        35                  40                  45
Gly Asp Arg Glu Val Trp Gly Met Ala Arg Ala Ser Glu Asp Phe Phe
    50                  55                  60
Phe Ile Leu Pro Val Thr Asp Asp Leu Thr Pro Val Leu Phe Tyr Asn
65                  70                  75                  80
Arg Leu Thr Asn Glu Pro Cys Phe Val Ser Asp Gln Gly Ile Thr Glu
                85                  90                  95
Tyr Phe Lys Phe Ala Gln Glu Gly Asp Tyr Ile Glu Val Glu Gly Ser
            100                 105                 110
Ser Val Phe Met Ala Asn Leu Leu Tyr Tyr Arg Phe Phe Pro Thr Arg
        115                 120                 125
Ile Thr Ser Tyr Asn Ala Pro Ile Glu Gly Val Val Ser Lys Thr Gly
    130                 135                 140
Asn Pro Ala Phe Thr Ile Pro Met Leu Pro Gly Val Ser Asp Cys Ile
145                 150                 155                 160

Glu Ile Ser Asn Asn Arg Lys Val Phe Leu Thr Asn Gln Leu Gly Val
                165                 170                 175
Val Asn Ile Thr Asp Gly Met Glu Pro Pro Ile Ile Ala Gly Val Ser
            180                 185                 190
Ala Ser Tyr Gly Ser Ser Val Arg Val Tyr Gly His Val Ser Gln Arg
        195                 200                 205
Trp Asp Ile Ile Gly His Cys Tyr Leu Asp Ile Tyr Pro Thr Asn Cys
    210                 215                 220
Tyr Pro Leu Ser Thr Lys Pro Val Ala Gly Asp Asp Glu Val Phe Val
225                 230                 235                 240
Lys Gln Gln Gly Arg Gln Ile Glu Ile Asp Ser Asn Ser Pro Ile Val
                245                 250                 255
Gln Val Val Val Tyr Asp Leu Glu Gly Lys Ser Val Phe Arg Lys Arg
            260                 265                 270
Met Thr Glu Asn Ala Tyr Thr Leu Ser Phe Arg Ala Pro Met Leu Gly
        275                 280                 285
Phe Met Thr Ile Met Ile Glu Thr Gln Asn Ser Ile Ile Asn Lys Lys
    290                 295                 300
Leu Asn Val Thr Gln Leu
305                 310

(2) INFORMATION FOR SEQ ID NO:528

      (i) SEQUENCE CHARACTERISTICS:
           (A) LENGTH: 405 amino acids
           (B) TYPE: amino acid
           (D) TOPOLOGY: linear

     (ii) MOLECULE TYPE: protein

    (iii) HYPOTHETICAL: YES

     (vi) ORIGINAL SOURCE:
           (A) ORGANISM: Porphyromonas gingivalis

     (ix) FEATURE:
           (A) NAME/KEY: misc feature
           (B) LOCATION 1...405

     (xi) SEQUENCE DESCRIPTION: SEQ ID NO:528

Met Lys Lys Thr Thr Ile Ile Ser Leu Ile Val Phe Gly Ala Phe Phe
1               5                   10                  15
Ala Ala Val Gly Gln Thr Lys Asp Asn Ser Ser Tyr Lys Pro Phe Ser
            20                  25                  30
Lys Glu Asp Ile Ala Gly Gly Val Tyr Ser Leu Pro Thr Gln Asn Arg
        35                  40                  45
Ala Gln Lys Asp Asn Ala Glu Trp Leu Leu Thr Ala Thr Val Ser Thr
    50                  55                  60
Asn Gln Ser Ala Asp Thr His Phe Ile Phe Asp Glu Asn Asn Arg Tyr
65                  70                  75                  80
Ile Ala Arg Asp Ile Lys Ala Asn Gly Val Arg Lys Ser Thr Asp Ser
                85                  90                  95
Ile Tyr Tyr Asp Ala Asn Gly Arg Ile Ser His Val Asp Leu Tyr Ile
            100                 105                 110
Ser Phe Ser Gly Gly Glu Pro Ala Leu Asp Thr Arg Phe Lys Tyr Thr
        115                 120                 125
Tyr Asp Asp Glu Gly Lys Met Thr Val Arg Glu Val Phe Met Leu Val
    130                 135                 140
Met Asp Pro Asn Thr Pro Ile Ser Arg Leu Glu Tyr His Tyr Asp Ala
145                 150                 155                 160
Gln Gly Arg Leu Thr His Trp Ile Ser Phe Ala Phe Gly Ala Glu Ser
                165                 170                 175
Gln Lys Asn Thr Tyr His Tyr Asn Glu Lys Gly Leu Leu Val Ser Glu
            180                 185                 190
Val Leu Ser Asn Ala Met Gly Thr Thr Tyr Ser Asp Thr Gly Lys Thr
        195                 200                 205
Glu Tyr Ser Tyr Asp Asp Ala Asp Asn Met Val Lys Ala Glu Tyr Phe
    210                 215                 220
Val Val Gln Gln Gly Lys Ala Trp Gln Val Leu Lys Arg Glu Glu Tyr
225                 230                 235                 240
Thr Tyr Glu Asp Asn Ile Cys Ile Gln Tyr Leu Ala Ile Asn Gly Thr
                245                 250                 255
Asp Thr Lys Val Tyr Lys Arg Asp Ile Glu Ser Asp Lys Ser Ile Ser
            260                 265                 270
Ala Asn Val Ile Asp Ile Pro Ser Met Pro Glu Gln Thr Trp Pro Asn
        275                 280                 285
Met Tyr Gly Phe Asn Ala Lys Arg Leu Lys Glu Thr Tyr Ser Ser Tyr
    290                 295                 300
Glu Gly Asp Val Ala Thr Pro Ile Phe Asp Tyr Ile Tyr Thr Tyr Lys
305                 310                 315                 320
Ala Leu Thr Ser Met Ala Thr Pro Ser Thr Glu Ala Gln Val Ala Val
                325                 330                 335
Tyr Leu Asn Pro Ser Thr Asp Arg Leu Val Ile Leu Ala Asn Gly Ile
            340                 345                 350
Thr His Leu Ser Met Tyr Asp Leu Gln Gly Lys Leu Ile Arg Asp Cys

355                 360                 365
Ala Leu Ser Gly Asp Lys Val Glu Met Gly Val Gly Ser Leu Thr Lys
    370                 375                 380
Gly Thr Tyr Leu Leu Lys Val Asn Thr Asp Gln Gly Ala Phe Val Arg
385                 390                 395                 400
Lys Val Val Ile Arg
                405

(2) INFORMATION FOR SEQ ID NO:529

     (i) SEQUENCE CHARACTERISTICS:
          (A) LENGTH: 2037 base pairs
          (B) TYPE: nucleic acid
          (C) STRANDEDNESS: double
          (D) TOPOLOGY: circular

    (ii) MOLECULE TYPE: DNA (genomic)

   (iii) HYPOTHETICAL: NO

    (iv) ANTI-SENSE: NO

    (vi) ORIGINAL SOURCE:
          (A) ORGANISM: Porphyromonas gingivalis

    (ix) FEATURE:
          (A) NAME/KEY: misc_feature
          (B) LOCATION 1...2037

    (xi) SEQUENCE DESCRIPTION: SEQ ID NO:529

GTAATGGACT ACAAACTCAC TTCTCGATTC AAGCCCACGG GCGACCAGCC GGAAGCCATT      60
CGCCAACTCG TACAGGGCAT CAACGAAGGG ATGCCGGCTC AGACGCTGCT CGGCGTAACG     120
GGTTCGGGCA AAACTTTTAC GGTGGCTAAC GTGGTGGCGG CGGTCAATCG TCCGACCCTT     180
GTCCTGAGTC ACAACAAGAC CTTGGCAGCA CAGCTATACG GAGAGTTCAA AGCCTTCTTC     240
CCCGAGAATG CGGTGGAGTA TTTCGTCAGC TACTACGACT ACTATCAGCC CGAGGCCTAC     300
CTCCCCGTCA CAGACACCTA TATCGAAAAG GACATGGCCA TCAACGCGGA GATCGAAAAA     360
CTGCGATTGA GGGCCACGGC TTCGCTCCTG TCAGGGCGGA AAGATGTGCT TGTGGTCAGC     420
TCCGTATCCT GTCTCTACGG TATGGCCAAT CCTGAAGCTT TTTCCGAAAA GGTGATCAGC     480
CTGCACACGG GACAAAGGGC AGACAGGGAT CATTTTATCC GCCTGCTGGT AGAGAGCTAC     540
TACACGAACA ATAAAGTAGA GTTCGAGAGC GGCAACTTCC GTGTCAAAGG CGACAGCGTG     600
GACATATTCC CCGCCGTAGA AGGTTATGAC GGCGTGGCAT ACAGGGTGGA GTTTTGGGAT     660
CCACACCTCG ACCGCCTGAC TACCTTCGAT CCCCGAACCC GACCCGAATA CGCCCTGCTC     720
TCGGAGCTGA AGATATATCC GGCCAATCTC TTCGTGACGA CTAAGGAGCA GGTGGATCGG     780
GCAGTAGGGA AAATCGATGT GGATCTGGGC GCACAGGTCG ATTTTCTGAA AGAAATCGGC     840
AAACCATATG AAGCCAAACG CTTGTATGAG CGGGTCACGT ATGACTTGGA AATGATCCGT     900
GAGTTGGGTT ATTGTTCCGG TATAGAGAAC TATTCGCGCT ACTTCGACGG CCGTGACGCG     960
GGCGAACGTC CTTTCTGTCT GTTGGATTAT TTCCCGGAGG ATTTCCTGTT GGTCATAGAC    1020
GAAAGCCATG TAACGATACC GCAGATACGT GCCATGTACG GAGGCGATCG TTCGCGCAAG    1080
GAGAATCTGG TCGAATACGG ATTCCGCCTG CCTGCCGCTC TCGACAATCG GCCGCTTCGC    1140
TTCGACGAGT TCGAAGCTCT CACCCCCCGG ACCCTTTATA TCAGTGCCAC GCCTGCCGAC    1200
TATGAGCTGA ACAGAAGCGA AGGCGTGATC GTCGAGCAGC TGATCCGTCC GACCGGACTG    1260
CTCCATCCCA TCATCCACCT CAAGCCCACC GCAAACCAAG TCGACGATCT GATGCACCAC    1320
ATAGCACGCT GCATCGAAAA GAAAGAGCGC GTACTGGTAA CGACCCTGAC CAAACGTATG    1380
GCAGAGGAGC TTAGCGAATA CCTGCTACGC CACGGTATCA GCACCGGCTA CATACACAGC    1440
GATGTGGACA CGCTGGAGCG TGTGCGTATC ATGGAAGACC TGCGCAAGGG GGTCTACGAT    1500
GCACTCATCG GGGTGAATCT GCTCCGCGAA GGATTGGACT TGCCGGAAGT TTCGCTTGTG    1560
GCTATTCTGG ATGCGGATAA GGAAGGATTC CTGCGCTCGC ATCGTTCGCT CACGCAGACT    1620
GCAGGACGTG CCGCCCGGCA CATTCATGGG CGTGTCATCT TCTACGCGGA CAAGATCACC    1680
CACACTATCC ACCTCACCAT CGACCACACT GCACCCCGAC GCCCAAACCA ACTCCCCTAC    1740
AACGAAGCGC ACGGCATCAC CCCCCAACAG ATAGTGAAGA ACAGTGCTGC CATTTGGGGA    1800
GAAGGCGATG TGTCGGCCTT GCAATCCGAT ACAGAATCCG GTGCGTACAT AGAAGAGAGC    1860
ACCATCGTCG CTCCCCATCC TTTGCCCCAC TATCTGACCA AACCCAACCT GCAACCACTC    1920
ATTGCTTCGA CCAAGAAGCA AATGCTGGCA GCAGCCAAAG AGCTGGACTT TCTGGAAGCG    1980
GCACGACTTC GGGACGAAGC CGCACGATTG GAAAAGAAAC TGGAGCAACT CACAGCC       2037

(2) INFORMATION FOR SEQ ID NO:530

     (i) SEQUENCE CHARACTERISTICS:
          (A) LENGTH: 2034 base pairs
          (B) TYPE: nucleic acid
          (C) STRANDEDNESS: double
          (D) TOPOLOGY: circular

    (ii) MOLECULE TYPE: DNA (genomic)

   (iii) HYPOTHETICAL: NO

    (iv) ANTI-SENSE: NO

    (vi) ORIGINAL SOURCE:
          (A) ORGANISM: Porphyromonas gingivalis

    (ix) FEATURE:
          (A) NAME/KEY: misc_feature

(B) LOCATION 1...2034

(xi) SEQUENCE DESCRIPTION: SEQ ID NO:530

ATGGACTACA AACTCACTTC TCGATTCAAG CCCACGGGCG ACCAGCCGGA AGCCATTCGC 60
CAACTCGTAC AGGGCATCAA CGAAGGGATG CCGGCTCAGA CGCTGCTCGG CGTAACGGGT 120
TCGGGCAAAA CTTTTACGGT GGCTAACGTG GTGGCGGCGG TCAATCGTCC GACCCTTGTC 180
CTGAGTCACA ACAAGACCTT GGCAGCACAG CTATACGGAG AGTTCAAAGC CTTCTTCCCC 240
GAGAATGCGG TGGAGTATTT CGTCAGCTAC TACGACTACT ATCAGCCCGA GGCCTACCTC 300
CCCGTCACAG ACACCTATAT CGAAAAGGAC ATGGCCATCA ACGCGGAGAT CGAAAAACTG 360
CGATTGAGGG CCACGGCTTC GCTCCTGTCA GGGCGGAAAG ATGTGCTTGT GGTCAGCTCC 420
GTATCCTGTC TCTACGGTAT GGCCAATCCT GAAGCTTTTT CCGAAAAGGT GATCAGCCTG 480
CACACGGGAC AAAGGGCAGA CAGGGATCAT TTTATCCGCC TGCTGGTAGA GAGCTACTAC 540
ACGAACAATA AAGTAGAGTT CGAGAGCGGC AACTTCCGTG TCAAAGGCGA CAGCGTGGAC 600
ATATTCCCCG CCGTAGAAGG TTATGACGGC GTGGCATACA GGGTGGAGTT TTGGGATGGA 660
GAGGTCGAGC GGCTGAGTAC CTTCGATCCG CGAACGGGAC GGGAATACGG CCTGCTGTCG 720
GAGCTGAAGA TATATCCGGC CAATCTCTTC GTGACGACTA AGGAGCAGGT GGATCGGGCA 780
GTAGGGAAAA TCGATGTGGA TCTGGGCGCA CAGGTCGATT TTCTGAAAGA AATCGGCAAA 840
CCATATGAAG CCAAACGCTT GTATGAGCGG GTCACGTATG ACTTGGAAAT GATCCGTGAG 900
TTGGGTTATT GTTCCGGTAT AGAGAACTAT TCGCGCTACT TCGACCGCCG TGACGCGGGC 960
GAACGTCCTT TCTGTCTGTT GGATTATTTC CCGGAGGATT TCCTGTTGGT CATAGACGAA 1020
AGCCATGTAA CGATACCGCA GATACGTGCC ATGTACGGAG GCGATCGTTC GCGCAAGGAG 1080
AATCTGGTCG AATACGGATT CCGCCTGCCT GCCGCTCTCG ACAATCGGCC GCTTCGCTTC 1140
GACGAGTTCG AAGCTCTCAC CCCCCGGACC CTTTATATCA GTGCCACGCC TGCCGACTAT 1200
GAGCTGAACA GAAGCGAAGG CGTGATCGTC GAGCAGCTGA TCCGTCCGAC CGGACTGCTG 1260
GATCCCATCA TCGACGTCAA GCCGACGGCA AACCAAGTGG ACGATCTGAT GGAGGAGATA 1320
GCACGCTGCA TCGAAAAGAA AGAGCGCGTA CTGGTAACGA CCCTGACCAA ACGTATGGCA 1380
GAGGAGCTTA GCGAATACCT GCTACGCCAC GGTATCAGCA CCGGCTACAT ACACAGCGAT 1440
GTGGACACGC TGGAGCGTGT GCGTATCATG GAAGACCTGC GCAAGGGGGT CTACGATGCA 1500
CTCATCGGGG TGAATCTGCT CCGCGAAGGA TTGGACTTGC CGGAAGTTTC GCTTGTGGCT 1560
ATTCTGGATG CGGATAAGGA AGGATTCCTG CGCTCGCATC GTTCGCTCAC GCAGACTGCA 1620
GGACGTGCCG CCCGGCACAT TCATGGGCGT GTCATCTTCT ACGCGGACAA GATCACCGAC 1680
ACTATGCACC TCACCATCCA CGACACTCCA CCCCCACGCC CAAACCAACT CGCCTACAAC 1740
GAAGCGCACG GCATCACCCC CCAACAGATA GTGAAGAACA GTGCTGCCAT TTGGGGAGAA 1800
GGCGATGTGT CGGCCTTGCA ATCCGATACA GAATCCGGTG CGTACATAGA AGAGAGCAGC 1860
ATGGTGGCTG CCGATCCTTT GGCCGACTAT CTGAGCAAAC CCAAGCTGGA AGCACTCATT 1920
GCTTCGACCA AGAAGCAAAT GCTGGCAGCA GCCAAAGAGC TGGACTTTCT GGAAGCGGCA 1980
CGACTTCGGG ACGAAGCCGC ACGATTGGAA AAGAAACTGG AGCAACTCAC AGCC 2034

(2) INFORMATION FOR SEQ ID NO:531

(i) SEQUENCE CHARACTERISTICS:
(A) LENGTH: 679 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...679

(xi) SEQUENCE DESCRIPTION: SEQ ID NO:531

Val Met Asp Tyr Lys Leu Thr Ser Arg Phe Lys Pro Thr Gly Asp Gln
1               5                   10                  15
Pro Glu Ala Ile Arg Gln Leu Val Gln Gly Ile Asn Glu Gly Met Pro
            20                  25                  30
Ala Gln Thr Leu Leu Gly Val Thr Gly Ser Gly Lys Thr Phe Thr Val
        35                  40                  45
Ala Asn Val Val Ala Ala Val Asn Arg Pro Thr Leu Val Leu Ser His
    50                  55                  60
Asn Lys Thr Leu Ala Ala Gln Leu Tyr Gly Glu Phe Lys Ala Phe Phe
65                  70                  75                  80
Pro Glu Asn Ala Val Glu Tyr Phe Val Ser Tyr Tyr Asp Tyr Tyr Gln
                85                  90                  95
Pro Glu Ala Tyr Leu Pro Val Thr Asp Thr Tyr Ile Glu Lys Asp Met
            100                 105                 110
Ala Ile Asn Ala Glu Ile Glu Lys Leu Arg Leu Arg Ala Thr Ala Ser
        115                 120                 125
Leu Leu Ser Gly Arg Lys Asp Val Leu Val Val Ser Ser Val Ser Cys
    130                 135                 140
Leu Tyr Gly Met Ala Asn Pro Glu Ala Phe Ser Glu Lys Val Ile Ser
145                 150                 155                 160
Leu His Thr Gly Gln Arg Ala Asp Arg Asp His Phe Ile Arg Leu Leu
                165                 170                 175
Val Glu Ser Tyr Tyr Thr Asn Asn Lys Val Glu Phe Glu Ser Gly Asn
            180                 185                 190
Phe Arg Val Lys Gly Asp Ser Val Asp Ile Phe Pro Ala Val Glu Gly
        195                 200                 205

Tyr Asp Gly Val Ala Tyr Arg Val Glu Phe Trp Asp Gly Glu Val Glu
    210                 215                 220
Arg Leu Ser Thr Phe Asp Pro Arg Thr Gly Arg Glu Tyr Gly Leu Leu
225                 230                 235                 240
Ser Glu Leu Lys Ile Tyr Pro Ala Asn Leu Phe Val Thr Thr Lys Glu
                245                 250                 255
Gln Val Asp Arg Ala Val Gly Lys Ile Asp Val Asp Leu Gly Ala Gln
            260                 265                 270
Val Asp Phe Leu Lys Glu Ile Gly Lys Pro Tyr Glu Ala Lys Arg Leu
        275                 280                 285
Tyr Glu Arg Val Thr Tyr Asp Leu Glu Met Ile Arg Glu Leu Gly Tyr
    290                 295                 300
Cys Ser Gly Ile Glu Asn Tyr Ser Arg Tyr Phe Asp Gly Arg Asp Ala
305                 310                 315                 320
Gly Glu Arg Pro Phe Cys Leu Leu Asp Tyr Phe Pro Glu Asp Phe Leu
                325                 330                 335
Leu Val Ile Asp Glu Ser His Val Thr Ile Pro Gln Ile Arg Ala Met
            340                 345                 350
Tyr Gly Gly Asp Arg Ser Arg Lys Glu Asn Leu Val Glu Tyr Gly Phe
        355                 360                 365
Arg Leu Pro Ala Ala Leu Asp Asn Arg Pro Leu Arg Phe Asp Glu Phe
    370                 375                 380
Glu Ala Leu Thr Pro Arg Thr Leu Tyr Ile Ser Ala Thr Pro Ala Asp
385                 390                 395                 400
Tyr Glu Leu Asn Arg Ser Glu Gly Val Ile Val Glu Gln Leu Ile Arg
                405                 410                 415
Pro Thr Gly Leu Leu Asp Pro Ile Ile Asp Val Lys Pro Thr Ala Asn
            420                 425                 430
Gln Val Asp Asp Leu Met Glu Glu Ile Ala Arg Cys Ile Glu Lys Lys
        435                 440                 445
Glu Arg Val Leu Val Thr Thr Leu Thr Lys Arg Met Ala Glu Glu Leu
    450                 455                 460
Ser Glu Tyr Leu Leu Arg His Gly Ile Ser Thr Gly Tyr Ile His Ser
465                 470                 475                 480
Asp Val Asp Thr Leu Glu Arg Val Arg Ile Met Glu Asp Leu Arg Lys
                485                 490                 495
Gly Val Tyr Asp Ala Leu Ile Gly Val Asn Leu Leu Arg Glu Gly Leu
            500                 505                 510
Asp Leu Pro Glu Val Ser Leu Val Ala Ile Leu Asp Ala Asp Lys Glu
        515                 520                 525
Gly Phe Leu Arg Ser His Arg Ser Leu Thr Gln Thr Ala Gly Arg Ala
    530                 535                 540
Ala Arg His Ile His Gly Arg Val Ile Phe Tyr Ala Asp Lys Ile Thr
545                 550                 555                 560
Asp Ser Met Gln Leu Thr Met Asp Glu Thr Ala Arg Arg Arg Ala Lys
                565                 570                 575
Gln Leu Ala Tyr Asn Glu Ala His Gly Ile Thr Pro Gln Gln Ile Val
            580                 585                 590
Lys Asn Ser Ala Ala Ile Trp Gly Glu Gly Asp Val Ser Ala Leu Gln
        595                 600                 605
Ser Asp Thr Glu Ser Gly Ala Tyr Ile Glu Glu Ser Ser Met Val Ala
    610                 615                 620
Ala Asp Pro Leu Ala Asp Tyr Leu Ser Lys Pro Lys Leu Glu Ala Leu
625                 630                 635                 640
Ile Ala Ser Thr Lys Lys Gln Met Leu Ala Ala Ala Lys Glu Leu Asp
                645                 650                 655
Phe Leu Glu Ala Ala Arg Leu Arg Asp Glu Ala Ala Arg Leu Glu Lys
            660                 665                 670
Lys Leu Glu Gln Leu Thr Ala
        675

(2) INFORMATION FOR SEQ ID NO:532

     (i) SEQUENCE CHARACTERISTICS:
          (A) LENGTH: 678 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...678

    (xi) SEQUENCE DESCRIPTION: SEQ ID NO:532

Met Asp Tyr Lys Leu Thr Ser Arg Phe Lys Pro Thr Gly Asp Gln Pro
1               5                   10                  15
Glu Ala Ile Arg Gln Leu Val Gln Gly Ile Asn Glu Gly Met Pro Ala
            20                  25                  30
Gln Thr Leu Leu Gly Val Thr Gly Ser Gly Lys Thr Phe Thr Val Ala

35                  40                  45
Asn Val Val Ala Ala Val Asn Arg Pro Thr Leu Val Leu Ser His Asn
    50                  55                  60
Lys Thr Leu Ala Ala Gln Leu Tyr Gly Glu Phe Lys Ala Phe Phe Pro
65                  70                  75                  80
Glu Asn Ala Val Glu Tyr Phe Val Ser Tyr Tyr Asp Tyr Tyr Gln Pro
                85                  90                  95
Glu Ala Tyr Leu Pro Val Thr Asp Thr Tyr Ile Glu Lys Asp Met Ala
            100                 105                 110
Ile Asn Ala Glu Ile Glu Lys Leu Arg Leu Arg Ala Thr Ala Ser Leu
        115                 120                 125
Leu Ser Gly Arg Lys Asp Val Leu Val Val Ser Ser Val Ser Cys Leu
    130                 135                 140
Tyr Gly Met Ala Asn Pro Glu Ala Phe Ser Glu Lys Val Ile Ser Leu
145                 150                 155                 160
His Thr Gly Gln Arg Ala Asp Arg Asp His Phe Ile Arg Leu Leu Val
                165                 170                 175
Glu Ser Tyr Tyr Thr Asn Asn Lys Val Glu Phe Glu Ser Gly Asn Phe
            180                 185                 190
Arg Val Lys Gly Asp Ser Val Asp Ile Phe Pro Ala Val Glu Gly Tyr
        195                 200                 205
Asp Gly Val Ala Tyr Arg Val Glu Phe Trp Asp Gly Glu Val Glu Arg
    210                 215                 220
Leu Ser Thr Phe Asp Pro Arg Thr Gly Arg Glu Tyr Gly Leu Leu Ser
225                 230                 235                 240
Glu Leu Lys Ile Tyr Pro Ala Asn Leu Phe Val Thr Thr Lys Glu Gln
                245                 250                 255
Val Asp Arg Ala Val Gly Lys Ile Asp Val Asp Leu Gly Ala Gln Val
            260                 265                 270
Asp Phe Leu Lys Glu Ile Gly Lys Pro Tyr Glu Ala Lys Arg Leu Tyr
        275                 280                 285
Glu Arg Val Thr Tyr Asp Leu Glu Met Ile Arg Glu Leu Gly Tyr Cys
    290                 295                 300
Ser Gly Ile Glu Asn Tyr Ser Arg Tyr Phe Asp Gly Arg Asp Ala Gly
305                 310                 315                 320
Glu Arg Pro Phe Cys Leu Leu Asp Tyr Phe Pro Glu Asp Phe Leu Leu
                325                 330                 335
Val Ile Asp Glu Ser His Val Thr Ile Pro Gln Ile Arg Ala Met Tyr
            340                 345                 350
Gly Gly Asp Arg Ser Arg Lys Glu Asn Leu Val Glu Tyr Gly Phe Arg
        355                 360                 365
Leu Pro Ala Ala Leu Asp Asn Arg Pro Leu Arg Phe Asp Glu Phe Glu
    370                 375                 380
Ala Leu Thr Pro Arg Thr Leu Tyr Ile Ser Ala Thr Pro Ala Asp Tyr
385                 390                 395                 400
Glu Leu Asn Arg Ser Glu Gly Val Ile Val Glu Gln Leu Ile Arg Pro
                405                 410                 415
Thr Gly Leu Leu Asp Pro Ile Ile Asp Val Lys Pro Thr Ala Asn Gln
            420                 425                 430
Val Asp Asp Leu Met Glu Glu Ile Ala Arg Cys Ile Glu Lys Lys Glu
        435                 440                 445
Arg Val Leu Val Thr Thr Leu Thr Lys Arg Met Ala Glu Glu Leu Ser
    450                 455                 460
Glu Tyr Leu Leu Arg His Gly Ile Ser Thr Gly Tyr Ile His Ser Asp
465                 470                 475                 480
Val Asp Thr Leu Glu Arg Val Arg Ile Met Glu Asp Leu Arg Lys Gly
                485                 490                 495
Val Tyr Asp Ala Leu Ile Gly Val Asn Leu Leu Arg Glu Gly Leu Asp
            500                 505                 510
Leu Pro Glu Val Ser Leu Val Ala Ile Leu Asp Ala Asp Lys Glu Gly
        515                 520                 525
Phe Leu Arg Ser His Arg Ser Leu Thr Gln Thr Ala Gly Arg Ala Ala
    530                 535                 540
Arg His Ile His Gly Arg Val Ile Phe Tyr Ala Asp Lys Ile Thr Asp
545                 550                 555                 560
Ser Met Gln Leu Thr Met Asp Glu Thr Ala Arg Arg Arg Ala Lys Gln
                565                 570                 575
Leu Ala Tyr Asn Glu Ala His Gly Ile Thr Pro Gln Gln Ile Val Lys
            580                 585                 590
Asn Ser Ala Ala Ile Trp Gly Glu Gly Asp Val Ser Ala Leu Gln Ser
        595                 600                 605
Asp Thr Glu Ser Gly Ala Tyr Ile Glu Glu Ser Ser Met Val Ala Ala
    610                 615                 620
Asp Pro Leu Ala Asp Tyr Leu Ser Lys Pro Lys Leu Glu Ala Leu Ile
625                 630                 635                 640
Ala Ser Thr Lys Lys Gln Met Leu Ala Ala Ala Lys Glu Leu Asp Phe
                645                 650                 655
Leu Glu Ala Ala Arg Leu Arg Asp Glu Ala Ala Arg Leu Glu Lys Lys
            660                 665                 670
Leu Glu Gln Leu Thr Ala
        675

## Claims

1. An isolated antigenic *Porphorymonas gingivalis* polypeptide, the polypeptide comprising;
an amino acid of SEQ. ID. NO. 518, SEQ. ID. NO. 519 or SEQ. ID. NO. 376; or
an amino acid sequence at least 85%, preferably at least 95%, identical to SEQ. ID. NO. 518, SEQ. ID. NO. 519 or SEQ. ID. NO. 376; or
at least 40 amino acids having a contiguous sequence of at least 40 amino acids identical to a contiguous amino acid sequence of SEQ. ID. NO. 518, SEQ. ID. NO. 519 or SEQ. ID. NO. 376.

2. An isolated DNA molecule, the DNA molecule comprising a nucleotide sequence which encodes the polypeptide as claimed in claim 1 or a sequence which hybridises thereto under conditions of high stringency.

3. An isolated DNA molecule as claimed in claim 2 in which the DNA molecule comprises a nucleotide sequence of SEQ. ID. NO. 112, SEQ. ID. NO. 254 or SEQ. ID. NO. 255.

4. A recombinant expression vector comprising the DNA molecule as claimed in claim 3 or claim 5 operably linked to a transcription regulatory element.

5. A cell comprising the recombinant expression vector as claimed in claim 4.

6. A method for producing a *P. gingivalis* polypeptide comprising culturing the cell as claimed in claim 5 under conditions that permit expression of the polypeptide.

7. A composition for use in raising an immune response directed against *P. gingivalis* in a subject, the composition comprising an effective amount of at least one polypeptide as claimed in claim 1 and a pharmaceutically acceptable carrier.

8. Use of a composition as claimed in claim 7 for treatment of *P. gingivalis* infection occurs.

9. The use as claimed in claim 8, wherein the treatment is a prophylactic treatment.

10. A composition for use in raising an immune response directed against *P. gingivalis* in a subject, the composition comprising an effective amount of at least one DNA molecule as claimed in claim 2 or claim 3 and a pharmaceutically acceptable carrier.

11. An antibody raised against a polypeptide as claimed in claim 1.

12. A composition comprising at least one antibody as claimed in claim 11.

13. A nucleotide probe comprising at least 18 nucleotides and having a contiguous sequence of at least 18 nucleotides identical to a contiguous nucleotide sequence of SEQ. ID. NO. 112, SEQ. ID. NO. 254 or SEQ. ID. NO. 255.

14. A nucleotide probe as claimed in claim 13 in which the probe further comprises a detectable label.

15. A method for detecting the presence of *P. gingivalis* nucleic acid in a sample comprising:
(a) contacting a sample with the nucleotide probe as claimed in claim 13 or claim 14 under conditions in which a hybrid can form between the probe and a *P. gingivalis* nucleic acid in the sample; and
(b) detecting the hybrid formed in step (a), wherein detection of a hybrid indicates the presence of a *P. gingivalis* nucleic acid in the sample.
